# EUROPEAN PATENT SPECIFICATION

(11) **EP 4 324 420 B1**
(45) Date of publication and mention of the grant of the patent: **08.10.2025**
(21) Application number: 23190944.1
(22) Date of filing: 10.08.2023
(51) Int. Cl.: A61B 34/20, G01R 33/022, A61B 1/00, A61B 17/00, A61B 90/00, A61B 5/06, G01R 33/10, G01R 33/02, H01F 7/04

(54) **SYSTEM**
SYSTEM
SYSTÈME

(30) Priority: 17.08.2022 JP 2022130247
(43) Date of publication of application: 21.02.2024
(73) Proprietor: FUJIFILM Corporation, Tokyo 106-8620 (JP)
(72) Inventor: TSUTO, Satoru, Kanagawa, 2588538 (JP); FUKUSHIMA, Kimitake, Kanagawa, 2588538 (JP)
(74) Representative: Dehns Germany Partnerschaft mbB

(56) References cited:
- KR-A- 20140 042 138
- US-A1- 2003 107 367
- US-A1- 2021 251 695

## Description

### BACKGROUND OF THE INVENTION

### 1. Field of the Invention

The present invention relates to a system.

### 2. Description of the Related Art

JP2007-319547A discloses a rotary self-propelled endoscope system comprising an insertion part that is formed with a spiral shaped portion on a surface and comprises a spiral tube which is rotationally movable about a major axis, a driving unit that applies a rotational driving force about the major axis to the spiral tube, a detection device that detects a rotational driving state of the spiral tube, and a control device that receives input of a detection result of the detection device and controls the driving unit based on the detection result.

JP2006-523129A discloses a device for deciding a position of an elongated instrument that can be inserted into a cavity, the device comprising at least one sensor disposed in the vicinity of the elongated instrument, and at least one magnetic field source disposed in the vicinity of the sensor, in which the sensor further detects movement of the elongated instrument in a case in which the elongated instrument is moved forward or backward with respect to the sensor. Instrument detection systems are also disclosed in US 2021/251695 A1 and KR 2014 0042138 A.

### SUMMARY OF THE INVENTION

The present disclosure provides a technology that makes it possible to easily determine a movement state of an instrument having an elongated shape.

The present disclosure relates to a system as defined by claim 1.

According to the present disclosure, it is possible to easily determine the movement state of the instrument having the elongated shape.

### BRIEF DESCRIPTION OF THE DRAWINGS

Fig. 1 is a diagram showing a schematic configuration of an endoscope system 200.
Fig. 2 is a partial cross-sectional view showing a detailed configuration of a flexible portion 10A of an endoscope 1.
Fig. 3 is a schematic diagram showing details of a magnetic pattern formed on a tubular member 17.
Fig. 4 is a schematic cross-sectional view taken along each of an A-A arrow and a B-B arrow in Fig. 3.
Fig. 5 is an exploded perspective view showing a configuration example of a detection unit 40.
Fig. 6 is a schematic diagram of a body part 42A of an insertion assisting member 40 shown in Fig. 5 as viewed from a direction x.
Fig. 7 is a diagram showing an example of a position at which the insertion part 10 can be located in a through-hole 41.
Fig. 8 is a schematic diagram showing an example of a magnetic flux density detected by a magnetic detection unit 43.
Fig. 9 is a schematic diagram showing an example of a result of classifying the magnetic flux density shown in Fig. 8 according to magnitude thereof.
Fig. 10 is a schematic diagram showing another example of the result of classifying the magnetic flux density shown in Fig. 8 according to the magnitude thereof.
Fig. 11 is a schematic cross-sectional view showing a modification example of magnetic pole portions MA1 and MA2 shown in Fig. 3 taken along the A-A arrow and the B-B arrow.
Fig. 12 is a schematic diagram showing an example of the magnetic flux density detected by the magnetic detection unit 43 from the tubular member 17 including the magnetic pole portions MA1 and MA2 shown in Fig. 11.
Fig. 13 is a schematic diagram showing a modification example of the magnetic pole portions MA1 and MA2 shown in Fig. 3.
Fig. 14 is a schematic diagram showing a magnetizer 60.
Fig. 15 is a schematic cross-sectional view showing a modification example of the magnetic pole portions MA1 and MA2 shown in Fig. 3 taken along the A-A arrow and the B-B arrow.
Fig. 16 is a diagram schematically showing a magnetic flux line generated in the magnetic pole portion MA1 having the configuration shown in Fig. 15.
Fig. 17 is a schematic cross-sectional view showing another modification example of the magnetic pole portions MA1 and MA2 shown in Fig. 3 taken along the A-A arrow and the B-B arrow.
Fig. 18 is a perspective view schematically showing a configuration example in which the detection unit 40 and the insertion assisting member are integrated.
Fig. 19 is a schematic cross-sectional view taken along an E-E arrow in Fig. 18.

### DESCRIPTION OF THE PREFERRED EMBODIMENTS

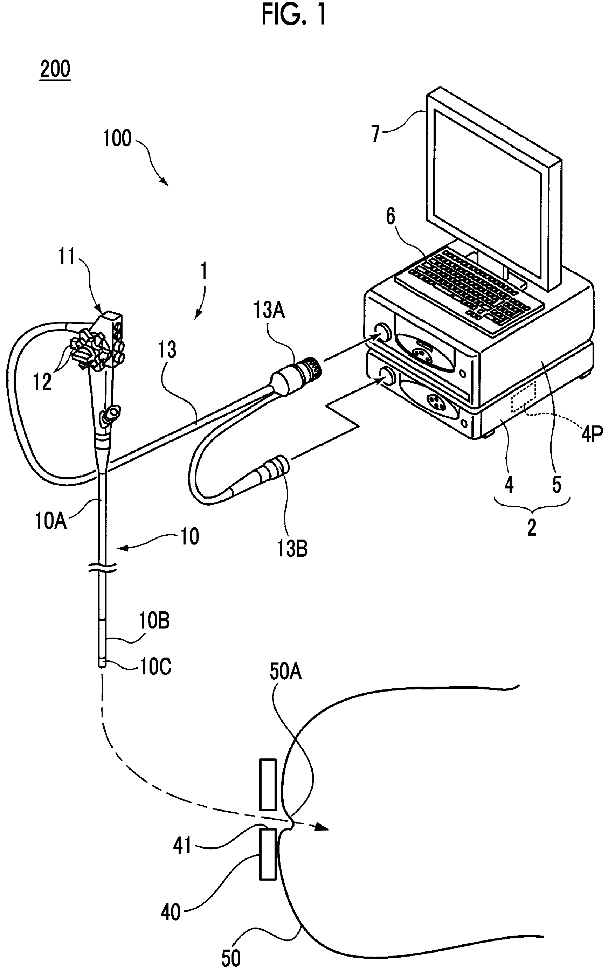

Fig. 1 is a diagram showing a schematic configuration of an endoscope system 200. The endoscope system 200 comprises an endoscope apparatus 100 including an endoscope 1, which is an example of a medical device that is inserted into a body and used for examination, surgery, or the like, and an insertion assisting member 40 that assists the insertion of the endoscope 1 into a body of a subject 50.

The endoscope 1 comprises an insertion part 10 which is an instrument having an elongated shape and extending in one direction and is inserted into the body, an operating part 11 provided in a base end portion of the insertion part 10 and provided with an operation member for performing an observation mode switching operation, an imaging recording operation, a forceps operation, an air supply/water supply operation, a suction operation, an electric cautery operation, or the like, an angle knob 12 provided adjacent to the operating part 11, and a universal cord 13 including connector portions 13A and 13B that connect the endoscope 1 to a light source device 5 and a processor device 4, in an attachable and detachable manner, respectively.

The operating part 11 is provided with a forceps port into which a biopsy forceps, which is a treatment tool for collecting a biological tissue, such as a cell or a polyp, is inserted. It should be noted that, although the illustration is omitted in Fig. 1, the inside of the operating part 11 and the insertion part 10, various channels are provided, such as a forceps channel through which the biopsy forceps inserted into the forceps port are inserted, a channel for air supply and water supply, a channel for suction, and the like.

The insertion part 10 includes a flexible portion 10A having flexibility, a bendable portion 10B provided at a distal end of the flexible portion 10A, and a distal end portion 10C that is harder than the flexible portion 10A and is provided at a distal end of the bendable portion 10B. An imaging element and an imaging optical system are built in the distal end portion 10C.

The bendable portion 10B is configured to be bendable by a rotational movement operation of the angle knob 12. Depending on a site of the subject in which the endoscope 1 is used, the bendable portion 10B can be bent in any direction and at any angle, and the distal end portion 10C can be directed in a desired direction.

Hereinafter, a direction in which the insertion part 10 extends will be referred to as a longitudinal direction X. In addition, one of radial directions of the insertion part 10 will be referred to as a radial direction Y. In addition, one of circumferential directions of the insertion part 10 (one of tangential directions of an outer peripheral edge of the insertion part 10) will be referred to as a circumferential direction Z. In the longitudinal direction X, a direction from a base end (operating part 11 side) of the endoscope 1 toward a distal end will be referred to as a longitudinal direction X1, and a direction from the distal end of the endoscope 1 to the base end will be referred to as a longitudinal direction X2. In addition, in the radial direction Y, one side will be referred to as a radial direction Y1 and the other side will be referred to as a radial direction Y2. The longitudinal direction X is one of directions different from the radial direction Y and the circumferential direction Z. The radial direction Y is one of directions different from the longitudinal direction X and the circumferential direction Z. In the present specification, the longitudinal direction X constitutes a first direction. Also, the radial direction Y constitutes a second direction intersecting the first direction. Further, the circumferential direction Z constitutes a third direction different from the first direction and the second direction.

In the example of Fig. 1, the insertion part 10 of the endoscope 1 is inserted into the body of the subject 50 from an anus 50A of the subject 50. The detection unit 40 has a rectangular plate shape as an example, and has a through-hole 41 into which the insertion part 10 can be inserted. The detection unit 40 is disposed between buttocks of the subject 50 and the insertion part 10 (that is, a movement path of the insertion part 10). The insertion part 10 reaches the anus 50A through the through-hole 41 of the detection unit 40, and is inserted into the body of the subject 50 from the anus 50A. In the present specification, the insertion part 10 constitutes an instrument that has an elongated shape and is used by being relatively moved with respect to the detection unit 40.

The endoscope apparatus 100 comprises the endoscope 1, a body part 2 consisting of the processor device 4 and the light source device 5 connected to the endoscope 1, a display unit 7 that displays a captured image and the like, and an input unit 6 which is an interface for inputting various pieces of information to the processor device 4.

The processor device 4 includes various processors 4P that control the endoscope 1, the light source device 5, and the display unit 7. The processor 4P is a central processing unit (CPU), which is a general-purpose processor that executes software (program including a display control program) to perform various functions, a programmable logic device (PLD), which is a processor of which a circuit configuration can be changed after the manufacture, such as a field programmable gate array (FPGA), and a dedicated electric circuit, which is a processor having a circuit configuration specially designed for executing specific processing, such as an application specific integrated circuit (ASIC). The processor 4P may be composed of one processor or a combination of two or more processors of the same type or a different type (for example, a plurality of FPGAs or a combination of a CPU and an FPGA). More specifically, the hardware structure of the processor 4P is an electric circuit (circuitry) in which circuit elements, such as semiconductor elements, are combined. In the present specification, the system is composed of the processor 4P, the insertion part 10 of the endoscope 1, and the detection unit 40.

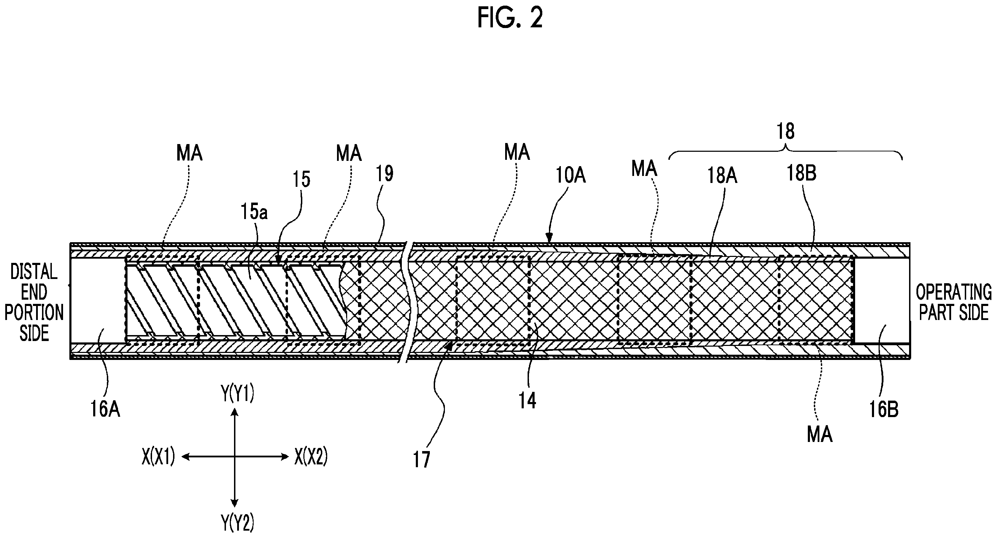

Fig. 2 is a partial cross-sectional view showing a detailed configuration of the flexible portion 10A of the endoscope 1. The flexible portion 10A, which forms most of a length of the insertion part 10, has flexibility over substantially the entire length thereof, and in particular, a portion to be inserted into a body cavity or the like has a structure that is rich in flexibility.

The flexible portion 10A comprises an outer skin layer 18 that constitutes a tubular member having an insulating property, and the tubular member 17 that is provided in the outer skin layer 18. The outer skin layer 18 is coated with a coating layer 19.

The tubular member 17 comprises a first member 14 that has a tubular shape, contains metal, and is coated with the outer skin layer 18, and a second member 15 that has a tubular shape, contains metal, and inserted into the first member 14. In the example of Fig. 2, the second member 15 is formed of a spiral tube formed by spirally winding a metal strip 15a. Also, the first member 14 is formed of a tubular-shaped net body formed by braiding a metal wire. The first member 14 and the second member 15 that continuously extend in the longitudinal direction X and have a thin structure are formed by plastic processing, and the metal constituting the first member 14 and the second member 15 includes austenite stainless steel. The austenite stainless steel cannot be magnetized in a state in which the plastic processing is not performed, but can be magnetized by performing the plastic processing. As described above, each of the first member 14 and the second member 15 constitutes a member that extends in the longitudinal direction X and contains metal.

The outer skin layer 18 is made of, for example, a resin such as an elastomer, and has a multi-layer structure of an inner resin layer 18A and an outer resin layer 18B. The outer skin layer 18 may have a monolayer structure. In the first member 14 and the second member 15, a cap 16A is fitted to an end portion on the distal end portion 10C side, and a cap 16B is fitted to an end portion on the operating part 11 side. The cap 16A and the cap 16B are covered with the outer skin layer 18. The flexible portion 10A is connected to the bendable portion 10B at the cap 16A and is connected to the operating part 11 at the cap 16B.

The tubular member 17 of the flexible portion 10A is formed with a magnetic pattern along the longitudinal direction X. The magnetic pattern along the longitudinal direction X refers to a pattern in which two types of magnetic pole regions, which are a negative pole (S pole) and a positive pole (N pole), are arranged in the longitudinal direction X in a predetermined arrangement pattern. As shown in Fig. 2, each of the first member 14 and the second member 15 is provided with a plurality of magnetic pole portions MA including the magnetic pole region.

At least one of the two types of magnetic pole regions, which are the negative pole (S pole) and the positive pole (N pole), is formed on the magnetic pole portion MA. As described above, each of the first member 14 and the second member 15 constitutes the member that extends in the longitudinal direction X and has the magnetic pattern formed along the longitudinal direction X.

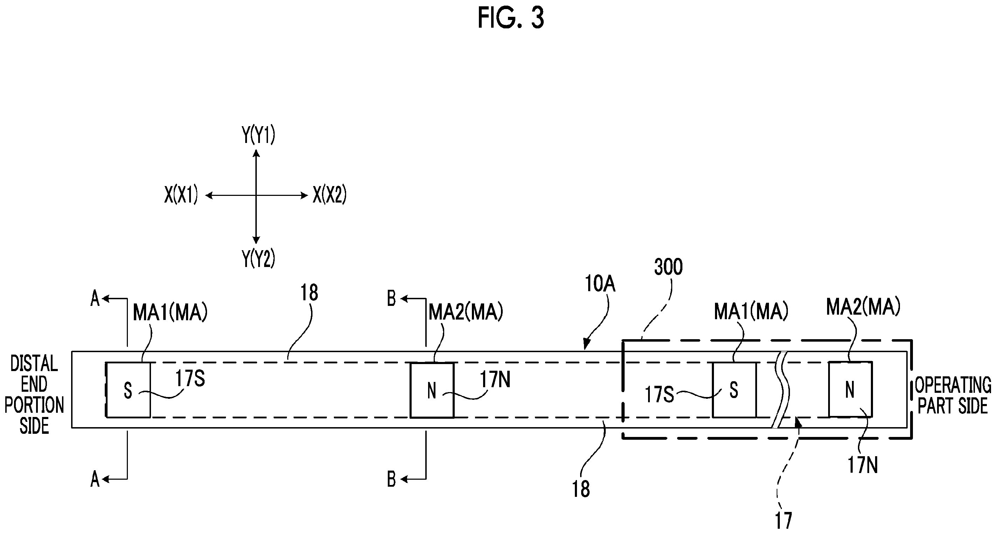

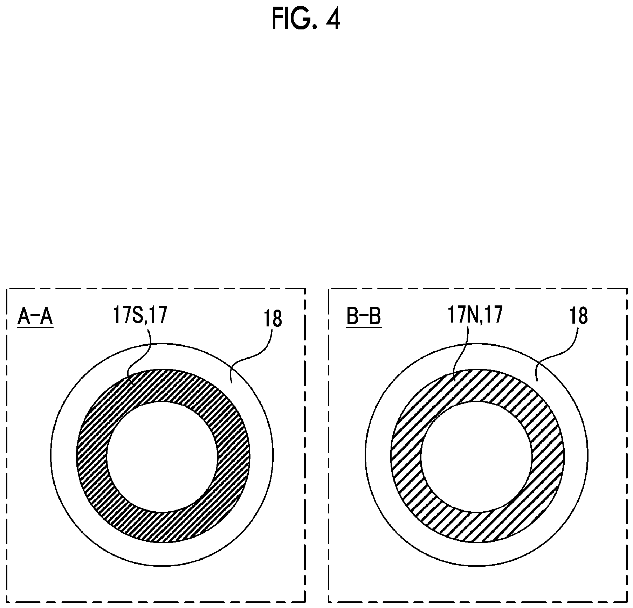

Fig. 3 is a schematic diagram showing details of the magnetic pattern formed on the tubular member 17. Fig. 4 is a schematic cross-sectional view taken along each of an A-A arrow and a B-B arrow in Fig. 3. As shown in Figs. 3 and 4, in the tubular member 17, a magnetic pole portion MA1 including a negative pole region 17S formed in an annular shape along the circumferential direction of the tubular member 17, and a magnetic pole portion MA2 including a positive pole region 17N formed in an annular shape along the circumferential direction of the tubular member 17 are provided to be alternately arranged in the longitudinal direction X. The total number of the magnetic pole portions MA1 and the total number of the magnetic pole portions MA2 are the same.

Here, an example of a manufacturing method of the endoscope 1 including the tubular member 17 having the magnetic pattern shown in Fig. 3 will be described. First, the endoscope 1 having the configuration shown in Fig. 1 is manufactured by a well-known method. Next, a magnetic field generation device 300 which has a cylindrical coil and can generate a magnetic field in the cylindrical coil by passing a current through the cylindrical coil is prepared. Next, as shown in Fig. 3, the insertion part 10 of the endoscope 1 is inserted into the cylindrical coil of the magnetic field generation device 300 from the distal end side to relatively move the coil to a boundary portion between the operating part 11 and the flexible portion 10A. In this state, a step of passing an alternating current through the cylindrical coil of the magnetic field generation device 300 to form the magnetic field, and pulling out the insertion part 10 from the cylindrical coil of the magnetic field generation device 300 in the longitudinal direction X2 at a constant speed is performed. In this step, a magnetic force of the tubular member 17 generated by the plastic processing is removed, and the tubular member 17 is demagnetized.

It should be noted that, in this step, it is preferable to pull out the insertion part 10 until the bendable portion 10B and the distal end portion 10C pass through the cylindrical coil and to demagnetize the entire insertion part 10. That is, in the insertion part 10 of the endoscope 1, it is preferable that the bendable portion 10B and the distal end portion 10C are demagnetized. The demagnetization of a certain region means that a magnetic flux density detected from the region is equal to or less than the geomagnetism.

After demagnetizing at least the tubular member 17 (flexible portion 10A), a state in which the cylindrical coil of the magnetic field generation device 300 is disposed on an outer periphery of the flexible portion 10A at a predetermined position in the longitudinal direction X is formed, and the alternating current is passed through the cylindrical coil in that state to form the magnetic field. By this work, the negative pole region 17S and the positive pole region 17N are formed over the entire circumferential direction of the tubular member 17 at positions in the vicinity of both ends of the cylindrical coil of the magnetic field generation device 300. Thereafter, by repeating this work while shifting the position of the flexible portion 10A with respect to the cylindrical coil in the longitudinal direction X, the magnetic pattern shown in Fig. 3 can be formed on the tubular member 17.

By adopting such a manufacturing method, any magnetic pattern can be easily formed on the tubular member 17 of the flexible portion 10A even in the endoscope 1 having the existing configuration or the endoscope 1 that has already been sold. Also, by demagnetizing the tubular member 17 of the flexible portion 10A and then forming the magnetic pattern on the tubular member 17, the magnetic pattern having a desired magnetic force can be formed with high accuracy. Also, by forming the magnetic pole region by using the cylindrical coil, it is possible to form the magnetic pole region having a uniform magnetic force (magnetic flux density) over the entire outer periphery of the tubular member 17 in the magnetic pole portion MA. It should be noted that, in Fig. 3, a boundary line between each of the negative pole region 17S, the positive pole region 17N, and the other region in the tubular member 17 is shown, but this boundary line is shown for convenience and is invisible. It should be noted that, it is preferable that information on the magnetic pattern formed on the tubular member 17 is stored in a memory (for example, a memory provided in the processor device 4) accessible to the processor 4P. The information on the magnetic pattern includes information indicating positions of the two types of magnetic pole regions in the tubular member 17, information indicating an arrangement pitch of the two types of magnetic pole regions in the tubular member 17, information indicating a range in which the magnetic pole region is formed on the insertion part 10, information indicating a position of a demagnetized region in the insertion part 10, or the like. The demagnetized region in the insertion part 10 constitutes an adjacent region adjacent to the region in which the magnetic pattern is formed on the insertion part 10. It should be noted that the bendable portion 10B and the distal end portion 10C are demagnetized regions in the insertion part 10, but the bendable portion 10B and the distal end portion 10C need only be configured to be distinguishable from the region in which the magnetic pattern is formed, and it is not essential that the bendable portion 10B and the distal end portion 10C are demagnetized. For example, magnetization may be performed with a pattern or a magnetic force that is clearly different from the magnetic pattern.

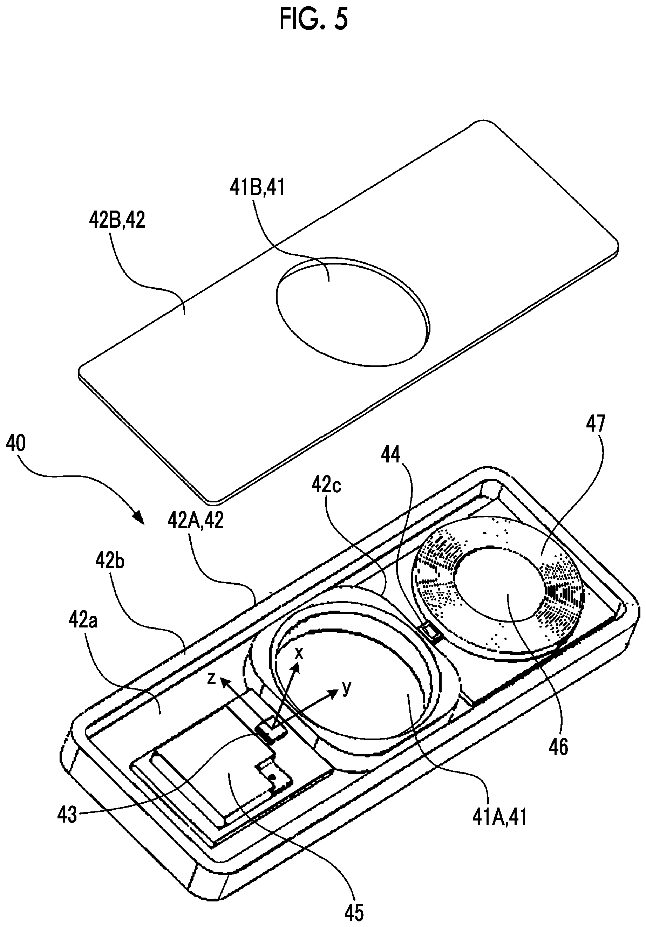

Fig. 5 is an exploded perspective view showing a configuration example of the detection unit 40. The detection unit 40 comprises a housing 42 having the through-hole 41, a magnetic detection unit 43 accommodated in the housing 42, a magnetic detection unit 44, a communication chip 45, a storage battery 46, and a power receiving coil 47.

The housing 42 comprises a body part 42A including a flat plate portion 42a that has rectangular flat plate shape and has a through-hole 41A penetrating in a thickness direction, a side wall portion 42b that has a rectangular frame shape that rises from an outer peripheral edge portion of the flat plate portion 42a in the thickness direction of the flat plate portion 42a, and an inner wall portion 42c that has a tubular shape that rises from a peripheral edge portion of the through-hole 41A in the flat plate portion 42a in the thickness direction of the flat plate portion 42a, and a lid part 42B that has a rectangular flat plate shape for closing an accommodation space surrounded by the flat plate portion 42a, the side wall portion 42b, and the inner wall portion 42c. The magnetic detection unit 43, the magnetic detection unit 44, the communication chip 45, the storage battery 46, and the power receiving coil 47 are accommodated in this accommodation space.

A through-hole 41B penetrating in the thickness direction is formed on the lid part 42B, and in a state in which the lid part 42B closes the accommodation space, the through-hole 41A and the through-hole 41B communicate with each other through an inner peripheral portion of the inner wall portion 42c to form the through-hole 41 into which the endoscope 1 can be inserted. It is preferable that the through-hole 41 has a perfect circular shape as viewed from an axial direction of the inner wall portion 42c (direction in which the endoscope 1 is inserted).

The housing 42 is preferably made of a resin or the like in order to reduce the weight and the cost, and preferably has a structure that prevents moisture from entering the accommodation space.

Each of the magnetic detection unit 43 and the magnetic detection unit 44 is disposed close to the inner wall portion 42c, and is a three-axis magnetic sensor that can detect a magnetic flux density in a direction x (direction along the axis of the through-hole 41) along the axis of the inner wall portion 42c, a magnetic flux density in a radial direction y of the through-hole 41, and a magnetic flux density in a direction z orthogonal to the direction x and the radial direction y.

In a state in which the insertion part 10 of the endoscope 1 is inserted into the through-hole 41, the longitudinal direction X and the direction x of the insertion part 10 match each other, and the radial direction Y and the radial direction y of the insertion part 10 match each other, and the circumferential direction Z of the insertion part 10 and the direction z match each other. Therefore, each of the magnetic detection unit 43 and the magnetic detection unit 44 is configured to detect a magnetic flux density BX in the longitudinal direction X of the insertion part 10, a magnetic flux density BY in the radial direction Y of the insertion part 10, and a magnetic flux density BZ in the circumferential direction Z of the insertion part 10. It should be noted that each of the magnetic detection unit 43 and the magnetic detection unit 44 may include three magnetic sensors, which are a uniaxial magnetic sensor that can detect the magnetic flux density BX, a uniaxial magnetic sensor that can detect the magnetic flux density BY, and a uniaxial magnetic sensor that can detect the magnetic flux density BZ. In the present specification, the magnetic flux density BX constitutes the first magnetic flux density, the magnetic flux density BY constitutes the second magnetic flux density, and the magnetic flux density BZ constitutes the third magnetic flux density.

Each of the magnetic detection unit 43 and the magnetic detection unit 44 need only be able to detect the magnetic flux density including the component in the longitudinal direction X, the magnetic flux density including the component in the radial direction Y, and the magnetic flux density including the component in the circumferential direction Z, and three detection axis directions and each of the longitudinal direction X, the radial direction Y, and the circumferential direction Z do not have to exactly match each other. In the magnetic sensor, in a case in which a first detection axis direction is different from the radial direction Y and the circumferential direction Z, a second detection axis direction is different from the longitudinal direction X and the circumferential direction Z, and a third detection axis direction is different from the radial direction Y and the longitudinal direction X, the magnetic sensor can detect the magnetic flux density including the component in the longitudinal direction X, can detect the magnetic flux density including the component in the radial direction Y, and can detect the magnetic flux density including the component in the circumferential direction Z.

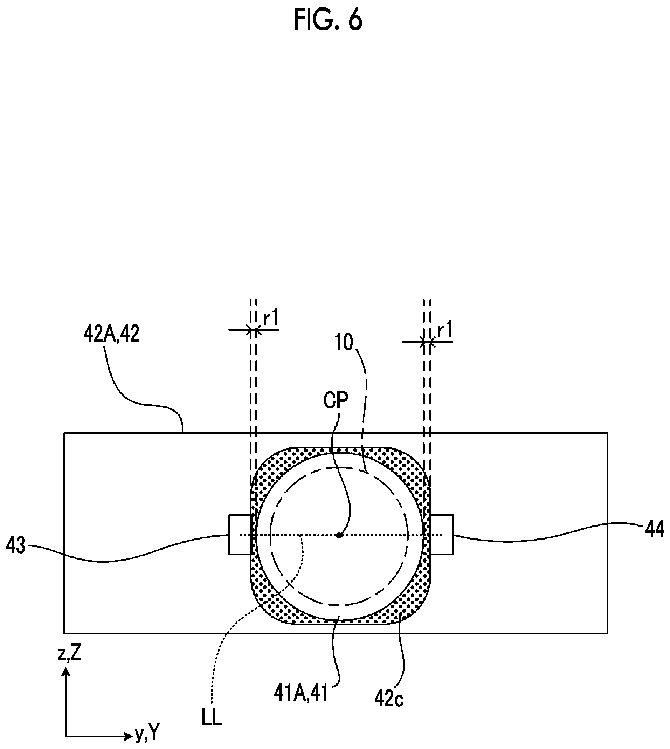

Fig. 6 is a schematic diagram of the body part 42A of the detection unit 40 shown in Fig. 5 as viewed from the direction x.

As shown in Fig. 6, the magnetic detection unit 43 and the magnetic detection unit 44 are disposed at positions facing each other with a center CP of the through-hole 41 interposed therebetween as viewed in the direction x. That is, in a state of being viewed in the direction x, a midpoint of a line segment LL connecting the magnetic detection unit 43 and the magnetic detection unit 44 and the center CP of the through-hole 41 substantially match each other. In other words, a distance from the magnetic detection unit 43 to the center CP of the through-hole 41 and a distance from the magnetic detection unit 44 to the center CP of the through-hole 41 substantially match each other.

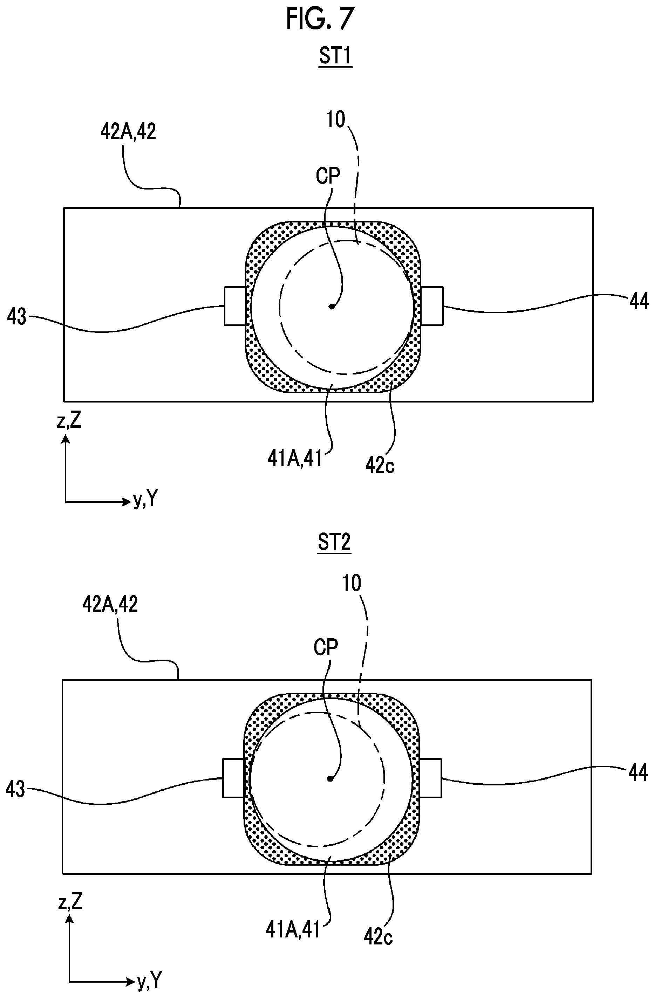

Fig. 7 is a diagram showing an example of a position at which the insertion part 10 can be located in the through-hole 41. A state ST1 of Fig. 7 shows a state in which the insertion part 10 is most distant from the magnetic detection unit 43 in the radial direction Y in the through-hole 41. A state ST2 of Fig. 7 shows a state in which the insertion part 10 is most distant from the magnetic detection unit 44 in the radial direction Y in the through-hole 41. A detection range and an installation position of each of the magnetic detection unit 43 and the magnetic detection unit 44 are determined such that the magnetic flux density can be detected with high accuracy from the magnetic pattern formed of the tubular member 17 in both the state ST1 or the state ST2 of Fig. 7.

In the present embodiment, as shown in Fig. 6, a thickness of a portion of the inner wall portion 42c in which the center CP and the position of the direction z are the same is a thickness r1. The thickness r1 is 0.5 mm, for example. In a case in which the magnetic force of the magnetic pole region formed on the tubular member 17 is defined by the magnetic flux density detected at a position distant from an outer surface of the insertion part 10 in the radial direction of the insertion part 10 by 0.5 mm, it is preferable that the magnetic force is made to a value that is sufficiently larger than the geomagnetism and is equal to or larger than a value (specifically, 500 microtesla) suitable for the performance of a general magnetic sensor. In addition, for example, in the state ST1 or the state ST2 of Fig. 7, it is preferable that the magnetic force of the magnetic pole region formed on the tubular member 17 is made to a range of 1000 microtesla to 1500 microtesla such that the magnetic detection unit 43 and the magnetic detection unit 44 can detect the magnetic flux density with high accuracy. It should be noted that an upper limit value of the magnetic force of the magnetic pole region formed on the tubular member 17 is preferably equal to or less than 20 millitesla such that the insertion part 10 does not adhere to another metal. In consideration of the maximum sensitivity of the general magnetic sensor, the upper limit value of the magnetic force of the magnetic pole region formed on the tubular member 17 is more preferably equal to or less than 2 millitesla.

As shown in Fig. 7, the position of the insertion part 10 can fluctuate in the through-hole 41. However, by obtaining the arithmetic mean of the magnetic flux density BX detected from the tubular member 17 by the magnetic detection unit 43 and the magnetic flux density BX detected from the tubular member 17 by the magnetic detection unit 44, it is possible to detect the magnetic flux density BX according to the magnetic pattern regardless of the position of the insertion part 10 in the through-hole 41. Similarly, by obtaining the arithmetic mean of the magnetic flux density BY detected from the tubular member 17 by the magnetic detection unit 43 and the magnetic flux density BY detected from the tubular member 17 by the magnetic detection unit 44, it is possible to detect the magnetic flux density BY according to the magnetic pattern regardless of the position of the insertion part 10 in the through-hole 41. Similarly, by obtaining the arithmetic mean of the magnetic flux density BZ detected from the tubular member 17 by the magnetic detection unit 43 and the magnetic flux density BZ detected from the tubular member 17 by the magnetic detection unit 44, it is possible to detect the magnetic flux density BZ according to the magnetic pattern regardless of the position of the insertion part 10 in the through-hole 41.

The communication chip 45 shown in Fig. 5 transmits information on the magnetic flux density detected by each of the magnetic detection unit 43 and the magnetic detection unit 44 to the processor device 4 by wireless communication. In the present specification, the communication chip 45 constitutes an output unit that outputs the information detected by the magnetic detection unit 43 and the magnetic detection unit 44 to the outside.

The storage battery 46 is charged by the power received by the power receiving coil 47 by the noncontact power supply. The magnetic detection unit 43, the magnetic detection unit 44, and the communication chip 45 are operated by the power supplied from the storage battery 46. The detection unit 40 includes a start-up switch (not shown). By performing an operation to turn on the start-up switch, the power is started to be supplied from the storage battery 46 to the magnetic detection unit 43, the magnetic detection unit 44, and the communication chip 45. It should be noted that the detection unit 40 may have a configuration in which the start-up switch is not provided and the power is started to be supplied to the magnetic detection unit 43, the magnetic detection unit 44, and the communication chip 45 by receiving wireless power supply from the outside. In a case in which the start-up switch is not provided, a structure in which the accommodation space of the housing 42 is completely sealed can be easily realized.

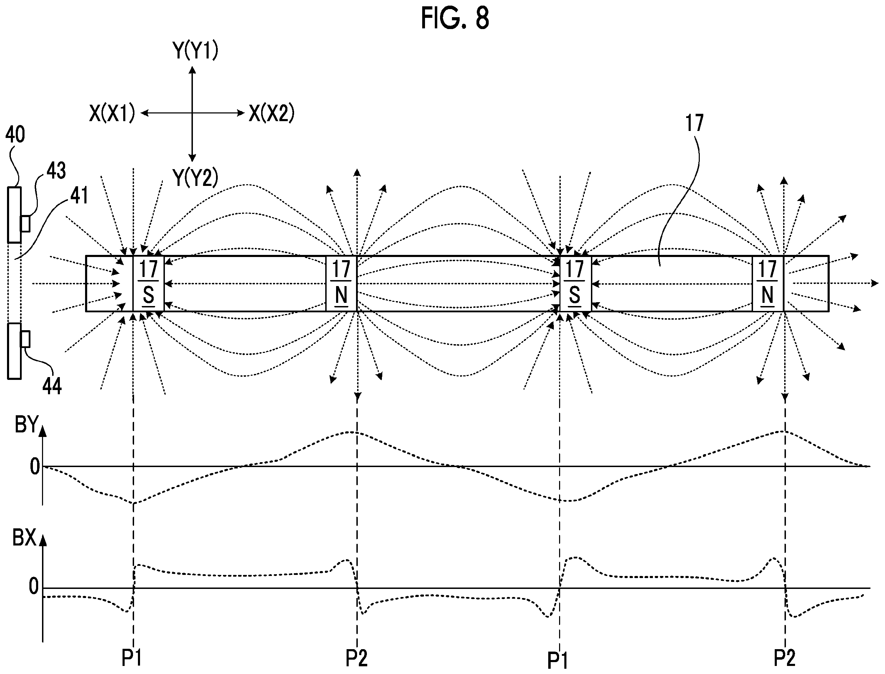

Fig. 8 is a schematic diagram showing an example of the magnetic flux density detected by the magnetic detection unit 43. It should be noted that, since the magnetic flux density detected by the magnetic detection unit 44 is the same as that in Fig. 8, the illustration is omitted. The two graphs shown in Fig. 8 show the magnetic flux density BX and the magnetic flux density BY detected by the magnetic detection unit 43 in a case in which the flexible portion 10A is moved in the longitudinal direction X1 through the through-hole 41. In Fig. 8, a magnetic flux line from the positive pole region 17N to the negative pole region 17S adjacent to the positive pole region 17N in the longitudinal direction X is indicated by a broken line arrow.

In a case in which the flexible portion 10A (tubular member 17) is moved toward the through-hole 41 of the detection unit 40 shown in the upper left of Fig. 8, as shown in the graph of Fig. 8, the magnetic flux density BX detected by the magnetic detection unit 43 has a positive value between each positive pole region 17N and the negative pole region 17S adjacent to the positive pole region 17N in the longitudinal direction X1, and has a negative value between each positive pole region 17N and the negative pole region 17S adjacent to the positive pole region 17N in the longitudinal direction X2. In addition, the magnetic flux density BY detected by the magnetic detection unit 43 has a negative value and a large absolute value in the vicinity of the negative pole region 17S, has a positive value and a large absolute value in the vicinity of the positive pole region 17N, and has a value close to zero in the vicinity of an intermediate position between the negative pole region 17S and the positive pole region 17N.

The magnetic flux densities detected from a plurality of positions in the longitudinal direction X of the tubular member 17 from the magnetic pattern formed on the tubular member 17 are magnetic flux densities in which the magnetic flux density BX and the magnetic flux density BY are respectively changed periodically with positive and negative values, and are magnetic flux densities in which the phases of the magnetic flux density BX and the magnetic flux density BY are shifted from each other in the longitudinal direction X. In the negative pole region 17S, an end (portion of a position P1 in Fig. 8) in the longitudinal direction X in which the absolute value of the magnetic flux density BY is maximum is hereinafter referred to as a negative pole end. In the positive pole region 17N, an end (portion of a position P2 in Fig. 8) in the longitudinal direction X in which the absolute value of the magnetic flux density BY is maximum is hereinafter referred to as a positive pole end.

As an example, by magnetizing the tubular member 17 by the method described above by setting a length of the cylindrical coil of the magnetic field generation device 300 in the axial direction is 60 mm, an inner diameter of the cylindrical coil of the magnetic field generation device 300 is 18 mm, and a movement pitch of the cylindrical coil in the longitudinal direction X is 144 mm, it is possible to form the magnetic pattern in which a distance between the negative pole end and the positive pole end is 72 mm. In the example of Fig. 8, for example, by disposing the cylindrical coil between the negative pole region 17S at the left end and the positive pole region 17N adjacent to the right side of the negative pole region 17S to form the magnetic field, it is possible to form these two magnetic pole regions. Then, from that state, by relatively moving the cylindrical coil by 144 mm in the longitudinal direction X2 to form the magnetic field in that state, it is possible to form the positive pole region 17N at the right end and the negative pole region 17S adjacent to the left side of the positive pole region 17N. In this manner, it is possible to form the magnetic pattern in which the distance (distance between the position P1 and the position P2) between the positive pole end and the negative pole end which are alternately formed in the longitudinal direction X is 72 mm.

In the endoscope system 200, the processor 4P of the processor device 4 acquires the information on the magnetic flux densities detected by the magnetic detection unit 43 and the magnetic detection unit 44 from the detection unit 40, and determines the movement state of the insertion part 10 in the longitudinal direction X based on the magnetic flux density BX and the magnetic flux density BY which are acquired. The movement state of the insertion part 10 determined here includes a movement direction indicating whether the insertion part 10 is moved with respect to the detection unit 40 in either direction along the longitudinal direction X, and a movement amount (movement distance) indicating a distance in which the insertion part 10 inserted into the through-hole 41 of the detection unit 40 is moved with respect to the detection unit 40 in the longitudinal direction X. It should be noted that the processor 4P obtains the arithmetic mean of the magnetic flux densities BX detected at the same timing by each of the magnetic detection unit 43 and the magnetic detection unit 44, obtains the arithmetic mean of the magnetic flux densities BY detected at the same timing by each of the magnetic detection unit 43 and the magnetic detection unit 44, and determines the movement state of the insertion part 10 based on the magnetic flux density BX and the magnetic flux density BY obtained by the arithmetic mean.

The processor 4P classifies the magnetic flux density BX into a plurality of pieces of information according to magnitude thereof, classifies the magnetic flux density BY into a plurality of pieces of information according to magnitude thereof, and determines the movement state of the insertion part 10 in the longitudinal direction X based on a combination of any of the plurality of pieces of information obtained by classifying the magnetic flux density BX and any of the plurality of pieces of information obtained by classifying the magnetic flux density BY.

Specifically, the processor 4P sets a first threshold value th (for example, "0") as a threshold value for classifying the magnetic flux density BX into two levels, and sets a second threshold value th1 (positive value larger than 0) and a second threshold value th2 (negative value less than 0) as a threshold value for classifying the magnetic flux density BY into three levels. Moreover, the processor 4P classifies the magnetic flux density BX by setting a value larger than the first threshold value th as a high level H and setting a value less than the first threshold value th as a low level L. Further, the processor 4P classifies the magnetic flux density BY setting a value larger than the second threshold value th1 as the high level H, setting a value between the second threshold value th1 and the second threshold value th2 as a middle level M, and setting a value less than the second threshold value th2 as the low level L. The result of classifying the magnetic flux density BX in this manner is also referred to as a classification level of the magnetic flux density BX, and the result of classifying the magnetic flux density BY in this manner is also referred to as a classification level of the magnetic flux density BY. In the present specification, among the classification levels of the magnetic flux density BX, the high level constitutes one of the fourth information or the fifth information, and the low level constitutes the other of the fourth information or the fifth information. In addition, among the classification levels of the magnetic flux density BY, the high level constitutes one of the first information or the second information, the low level constitutes the other of the first information or the second information, and the middle level constitutes the third information.

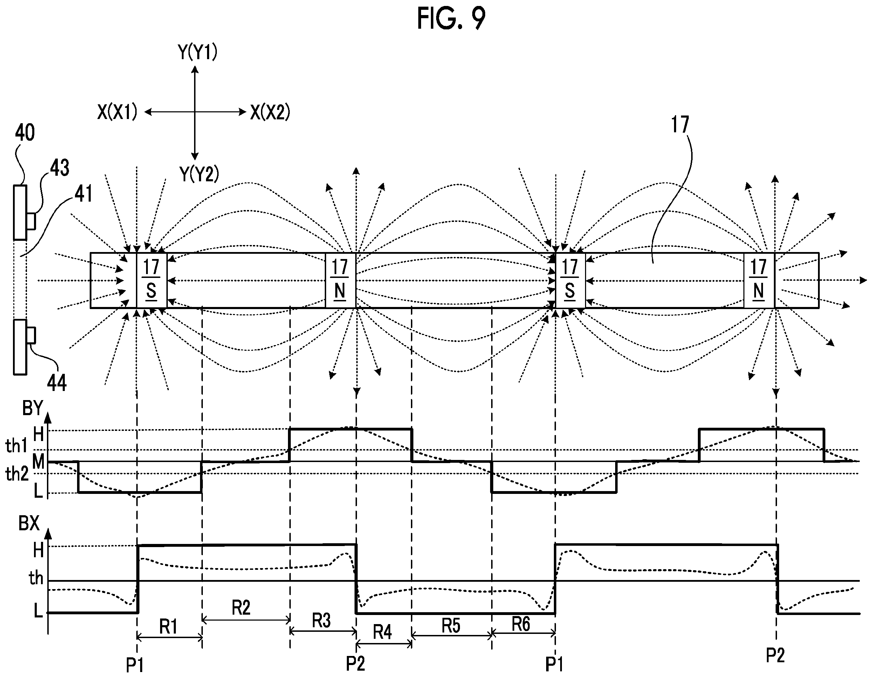

In Fig. 9, the result (classification level) of classifying the magnetic flux density BX and the magnetic flux density BY in the graph shown in Fig. 8 is shown by a thick solid line. As shown in Fig. 9, in the tubular member 17, a range between two adjacent positions P1 (between the negative pole ends) is divided into a region R1 in which the magnetic flux density BX is at the high level and the magnetic flux density BY is at the low level, a region R2 in which the magnetic flux density BX is at the high level and the magnetic flux density BY is at the middle level, a region R3 in which the magnetic flux density BX is at the high level and the magnetic flux density BY is at the high level, a region R4 in which the magnetic flux density BX is at the low level and the magnetic flux density BY is at the high level, a region R5 in which the magnetic flux density BX is at the low level and the magnetic flux density BY is at the middle level, and a region R6 in which the magnetic flux density BX is at the low level and the magnetic flux density BY is at the low level. As described above, the range between the negative pole ends adjacent to each other in the longitudinal direction X can be divided into six regions R1 to R6 depending on a combination of the classification level of the magnetic flux density BX and the classification level of the magnetic flux density BY.

By monitoring the thick solid lines (classification levels of the magnetic flux densities BX and BY) shown in Fig. 9, the processor 4P determines the movement direction of the insertion part 10 with respect to the detection unit 40 and the movement amount (movement distance) of the insertion part 10 in the longitudinal direction X starting from the position of the detection unit 40.

For example, in a case in which the negative pole region 17S provided on the most distal end side of the tubular member 17 passes through the through-hole 41, the processor 4P detects that the region R1 at the most distal end of the tubular member 17 is located in the through-hole 41 from the combination of the classification level of the magnetic flux density BX and the classification level of the magnetic flux density BY, and detects the position as a reference position. A distance (referred to as a distance L1) in the longitudinal direction X from the negative pole region 17S provided on the most distal end side of the tubular member 17 to the distal end of the distal end portion 10C is known. Therefore, in a case in which this reference position is detected, the processor 4P determines that the movement distance of the insertion part 10 with respect to the detection unit 40 is "0", and further determines that an insertion length of the insertion part 10 into the body of the subject 50 is the distance L1.

After the reference position is detected, in a case in which it is determined that the region of the tubular member 17 passing through the through-hole 41 is changed in a direction from the region R1 to the region R6 by the classification levels of the magnetic flux densities BX and BY, the processor 4P determines that the insertion part 10 is moved in the longitudinal direction X1. In addition, in a case in which it is determined that the insertion part 10 is moved in the longitudinal direction X1, the processor 4P increases the movement distance of the insertion part 10 in the longitudinal direction X1 by a unit distance ΔL and increases the insertion length of the insertion part 10 into the body of the subject 50 by the unit distance ΔL, each time the region of the tubular member 17 passing through the through-hole 41 is changed by one (for example, a change from the region R1 to the region R2 or a change from the region R2 to the region R3). The unit distance ΔL can be a value obtained by dividing the distance between the negative pole regions 17S adjacent to each other by 6.

On the other hand, in a case in which it is determined that the region of the tubular member 17 passing through the through-hole 41 is changed in a direction from the region R6 to the region R1 by the classification levels of the magnetic flux densities BX and BY, the processor 4P determines that the insertion part 10 is moved in the longitudinal direction X2. In addition, in a case in which it is determined that the insertion part 10 is moved in the longitudinal direction X2, the processor 4P decreases the movement distance of the insertion part 10 in the longitudinal direction X1 by a unit distance ΔL and decreases the insertion length of the insertion part 10 into the body of the subject 50 by the unit distance ΔL, each time the region of the tubular member 17 passing through the through-hole 41 is changed by one.

It should be noted that, depending on the movement speed of the insertion part 10, it can also be determined that the region of the tubular member 17 passing through the through-hole 41 is changed from the region R1 to the region R3 or is changed from the region R3 to the region R1. In a case in which it is determined that the region of the tubular member 17 passing through the through-hole 41 is changed by two in this manner, the processor 4P need only increase or decrease the insertion length of the insertion part 10 by twice the unit distance ΔL.

The processor 4P displays the information on the insertion length determined in this manner on the display unit 7, outputs the information by voice from a speaker (not shown), or transmits the information to an operator of the endoscope 1 by vibration of a vibrator provided in the operating part 11. As a result, it is possible to record an imaging position by the endoscope 1, guide or evaluate the operation of the endoscope 1, and the like with high accuracy.

It should be noted that, as described above, in the insertion part 10, the distal end portion 10C and the bendable portion 10B are demagnetized, so that the processor 4P can easily detect the reference position. Specifically, in a case in which the insertion part 10 is inserted into the through-hole 41 from the distal end side and is moved in the longitudinal direction X1, both the magnetic flux density BX and the magnetic flux density BY are values in the vicinity of "0" while the distal end portion 10C and the bendable portion 10B pass through the through-hole 41. Further, at a point in time at which the negative pole region 17S on the most distal end side of the tubular member 17 reaches the through-hole 41, the magnetic flux density BX and the magnetic flux density BY are a combination of the high level and the low level as shown in Fig. 9, so that it can be easily detect the reference position by the fluctuation of the magnetic flux density.

As described above, the processor 4P classifies the magnetic flux density BX into two which are the high level and the low level, classifies the magnetic flux density BY into three which are the high level, the middle level, and the low level, and determines the movement state of the insertion part 10 in the longitudinal direction X based on the combination thereof. In this way, by monitoring the change in the combination of the classification level of the magnetic flux density BX and the classification level of the magnetic flux density BY, the movement direction, the movement distance, and the insertion length of the insertion part 10 can be determined. With the endoscope system 200, such an effect can be realized only by magnetizing the endoscope 1 having a general-purpose configuration and adding the detection unit 40, so that a construction cost of the system can be reduced. In addition, since the movement direction, the movement distance, and the insertion length of the insertion part 10 are determined based on the information on the magnetic flux density that can be acquired non-optically, even in a case in which the insertion part 10 is dirty, the determination accuracy is not reduced, and thus it is practical.

In addition, by using the combination of the classification level of the magnetic flux density BX and the classification level of the magnetic flux density BY, it is possible to determine the movement distance of the insertion part 10 with a resolution (for example, a unit of 1/3 of the interval) finer than the interval between the two types of adjacent magnetic pole regions (negative pole region 17S and positive pole region 17N). In this way, the movement distance can be finely determined, which can be useful for accurate recording of the imaging position by the endoscope 1, guide or evaluation of the operation of the endoscope 1, and the like.

In addition, the processor 4P obtains the arithmetic mean of the magnetic flux density detected by the magnetic detection unit 43 and the magnetic flux density detected by the magnetic detection unit 44, and determines the movement direction, the movement distance, and the insertion length of the insertion part 10 based on the magnetic flux density of the arithmetic mean. Therefore, it is possible to obtain the change in the magnetic flux density according to the magnetic pattern regardless of the position of the insertion part 10 in the through-hole 41. In addition, the magnetic flux densities detected by the magnetic detection unit 43 and the magnetic detection unit 44 can include a disturbance component caused by geomagnetism, a magnetic field generated by a steel frame of a building, a magnetic field generated by the steel furniture, and the like, in addition to a magnetic field generated by magnetization. However, as described above, by obtaining the arithmetic mean of the magnetic flux densities detected by the two magnetic detection units, it is possible to reduce an influence of the disturbance component.

It should be noted that, in a case in which a difference between an inner diameter of the through-hole 41 and an outer diameter of the insertion part 10 is made as small as possible, any one of the magnetic detection unit 43 or the magnetic detection unit 44 provided in the detection unit 40 is not essential and can be omitted. In this case, the processor 4P need only determine the movement direction, the movement distance, and the insertion length of the insertion part 10 based on the magnetic flux density BX and BY detected by the magnetic detection unit 43 or the magnetic detection unit 44.

In addition, in the present embodiment, the negative pole region 17S and the positive pole region 17N formed on the tubular member 17 are each formed in an annular shape along an outer periphery of the tubular member 17. Therefore, even in a case in which the insertion part 10 is rotated in the circumferential direction thereof in the through-hole 41, it is possible to substantially eliminate the change in the magnetic flux densities detected by the magnetic detection unit 43 and the magnetic detection unit 44. Therefore, the movement direction, the movement distance, and the insertion length of the insertion part 10 can be determined regardless of the posture of the insertion part 10.

The disturbance component can be included in the magnetic flux densities detected by the magnetic detection unit 43 and the magnetic detection unit 44. In addition, a direction of the disturbance component is also changed depending on a posture of the detection unit 40. Therefore, rather than the determination of the movement state of the insertion part 10 in the longitudinal direction X using the raw data of the magnetic flux density BX and the magnetic flux density BY as they are, as described above, in a case in which the magnetic flux density BX is classified into two which are the high level and the low level, the magnetic flux density BY is classified into three which are the high level, the middle level, and the low level, and the movement state of the insertion part 10 in the longitudinal direction X is determined based on the combination of the classification levels, so that the influence of the disturbance component can be eliminated.

As described above, the processor 4P classifies the magnetic flux density BX into two which are the high level and the low level, classifies the magnetic flux density BY into three which are the high level, the middle level, and the low level, and determines the movement state of the insertion part 10 in the longitudinal direction X based on the combination of the classification levels. As a modification example, the processor 4P may classify the magnetic flux density BX into two which are the high level and the low level, may classify the magnetic flux density BY into two which are the high level and the low level, and may determine the movement state of the insertion part 10 in the longitudinal direction X based on the combination of the classification levels.

Specifically, the processor 4P sets the "first threshold value th (for example, 0)" as the threshold value for classifying the magnetic flux density BX into two levels, and sets a "second threshold value th3 (for example, 0)" as the threshold value for classifying the magnetic flux density BY into two levels. Moreover, the processor 4P classifies the magnetic flux density BX by setting a value larger than the first threshold value th as the high level and setting a value less than the first threshold value th as the low level. In addition, the processor 4P classifies the magnetic flux density BY setting a value larger than the second threshold value th3 as the high level and setting a value less than the second threshold value th3 as the low level.

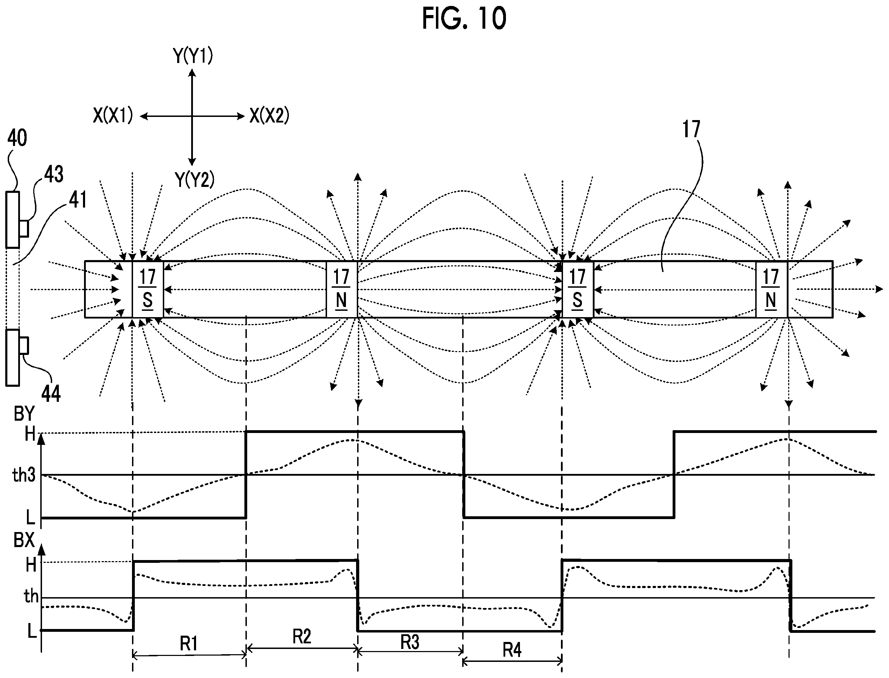

In Fig. 10, the result (classification level) of classifying the magnetic flux density BX and the magnetic flux density BY in the graph shown in Fig. 8 is shown by a thick solid line. As shown in Fig. 10, in the tubular member 17, a range between two adjacent positions P1 is divided into a region R1 in which the magnetic flux density BX is at the high level and the magnetic flux density BY is at the low level, a region R2 in which the magnetic flux density BX is at the high level and the magnetic flux density BY is at the high level, a region R3 in which the magnetic flux density BX is at the low level and the magnetic flux density BY is at the high level, and a region R4 in which the magnetic flux density BX is at the low level and the magnetic flux density BY is at the low level. As described above, the range between the negative pole ends adjacent to each other in the longitudinal direction X can be divided into four regions R1 to R4 depending on a combination of the classification level of the magnetic flux density BX and the classification level of the magnetic flux density BY. By monitoring the thick solid lines (classification levels of the magnetic flux densities BX and BY) shown in Fig. 10, the processor 4P can determine the movement direction of the insertion part 10 and the movement amount (movement distance) of the insertion part 10 in the longitudinal direction X.

It should be noted that, as described so far, the processor 4P classifies the magnetic flux density into the plurality of pieces of information according to the magnitude thereof. However, a configuration may be adopted in which this classification is performed by a processor provided in the communication chip of the detection unit 40. That is, a configuration may be adopted in which the detection unit 40 transmits information on the classification level shown by a thick solid line shown in Fig. 9 or Fig. 10 to the processor device 4. Also, the processor 4P of the processor device 4 performs the determination of the movement state of the insertion part 10, but a configuration may be adopted in which the processor provided in the communication chip of the detection unit 40 performs the determination to transmit the determination result to the processor device 4. In addition, a configuration may be adopted in which a processor, such as a personal computer connected to the processor device 4 via a network, acquires the information on the magnetic flux density from the detection unit 40, performs the determination, and transmits the determination result to the processor device 4. Also, a processor separately from the processor 4P provided in the processor device 4 may perform the determination of the movement state of the insertion part 10. Further, a configuration may be adopted in which a processor provided outside the endoscope apparatus 100 performs the determination of the movement state of the insertion part 10 to transmit the determination result to the processor device 4.

The threshold value used in a case of classifying each of the magnetic flux density BX and the magnetic flux density BY according to the magnitude thereof may be a predetermined fixed value, but the threshold value is preferably a fluctuation value determined based on the magnetic flux densities detected by the magnetic detection unit 43 and the magnetic detection unit 44 after the insertion of the insertion part 10 into the through-hole 41 is started.

For example, in a case in which the start-up switch of the detection unit 40 is turned on, the insertion part 10 is inserted into the through-hole 41, and the third magnetic pole region from the most distal end side of the tubular member 17 passes through the through-hole 41, the processor 4P can acquire the maximum value and the minimum value of the magnetic flux density BX detected by the magnetic detection unit 43, and the maximum value and the minimum value of the magnetic flux density BY detected by the magnetic detection unit 43, respectively. The processor 4P acquires the maximum value and the minimum value of the magnetic flux density BX, obtains an average value of the maximum value and the minimum value, and sets the average value as the first threshold value th. Further, in a case in which the maximum value and the minimum value of the magnetic flux density BY are acquired, the processor 4P obtains an average value of the maximum value and the minimum value, and sets a value obtained by adding a predetermined value to the average value as the second threshold value th1, and sets a value obtained by subtracting a predetermined value from the average value as the second threshold value th2. The predetermined value is a value that is larger than a value assumed as the disturbance component and less than the absolute values of the maximum value and the minimum value of the magnetic flux density BY. The first to third magnetic pole regions from the most distal end side of the tubular member 17 constitute a base end portion on the demagnetized region (adjacent region) side in the region in which the magnetic pattern is formed.

Hereinafter, the processor 4P need only classify the magnetic flux density BX and the magnetic flux density BY using the threshold values set in this manner. In this way, by setting the threshold values based on the magnetic flux densities detected by the magnetic detection unit 43 and the magnetic detection unit 44, it is possible to perform the determination of the movement state of the insertion part 10 with higher accuracy.

It should be noted that, in this way, in a case in which the threshold value is set based on the magnetic flux densities detected by the magnetic detection unit 43 and the magnetic detection unit 44, it is preferable that, in a period until the third magnetic pole region from the most distal end side of the tubular member 17 passes through the through-hole 41, the processor 4P sets the first threshold value th, the second threshold value th1, and the second threshold value th2 to the predetermined values, performs the detection of the reference position or the determination of the movement state of the insertion part 10, and then updates the first threshold value th, the second threshold value th1, and the second threshold value th2 by the method described above to perform the determination of the movement state of the insertion part 10.

As described above, in the endoscope system 200, the magnetic pattern is formed on the tubular member 17 such that the magnetic flux densities BX and BY detected by each of the magnetic detection unit 43 and the magnetic detection unit 44 are changed periodically between the positive side and the negative side and phases thereof are shifted in a case in which the insertion part 10 passes through the through-hole 41, so that it is possible to perform the determination of the movement state of the insertion part 10. Such a magnetic pattern is not limited to the configurations of the magnetic pole portions MA1 and MA2 shown in Figs. 3 and 4, and can be variously modified.

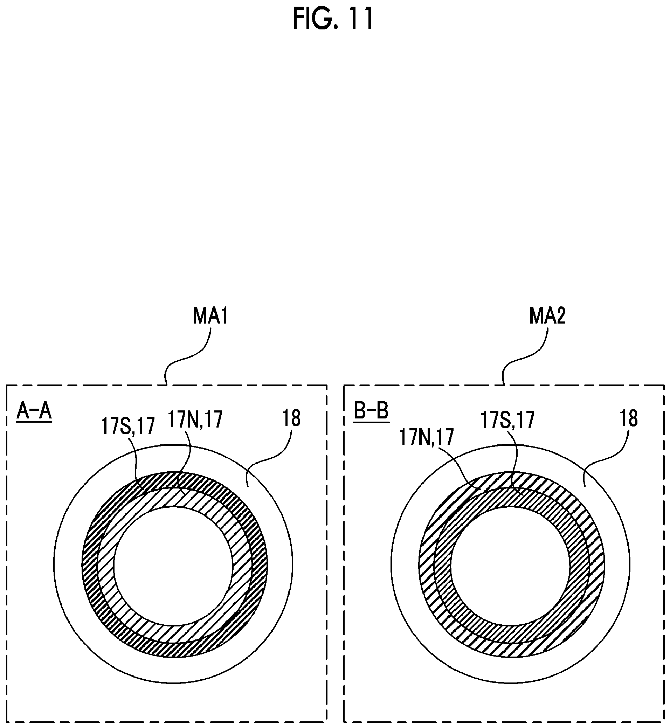

Fig. 11 is a schematic cross-sectional view showing a modification example of the magnetic pole portions MA1 and MA2 shown in Fig. 3 taken along the A-A arrow and the B-B arrow. In the modification example shown in Fig. 11, the magnetic pole portion MA1 has a configuration in which the negative pole region 17S having an annular shape is formed on an outer peripheral side and the positive pole region 17N having an annular shape is formed inside the negative pole region 17S. In addition, the magnetic pole portion MA2 has a configuration in which the positive pole region 17N having an annular shape is formed on the outer peripheral side, and the negative pole region 17S having an annular shape is formed inside the positive pole region 17N.

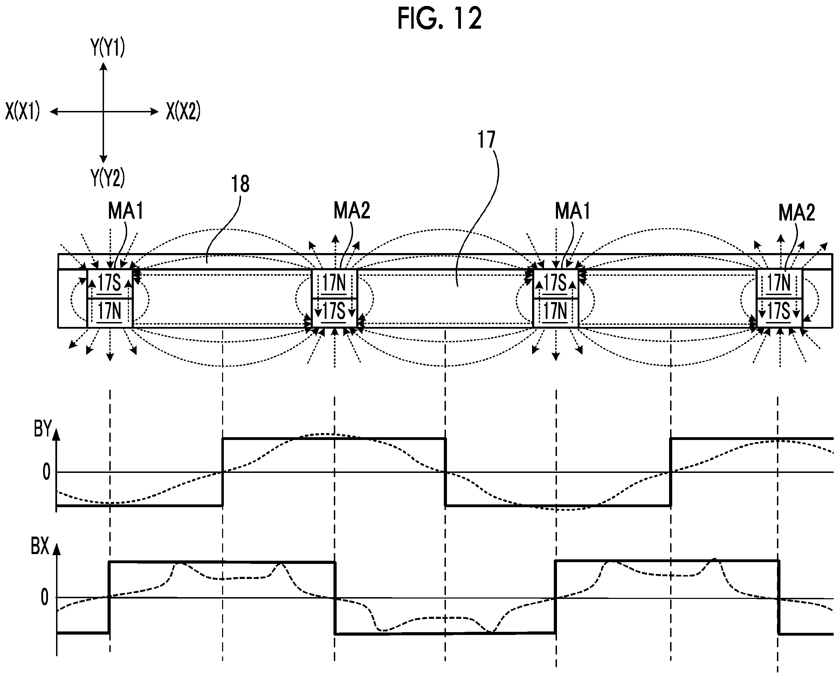

Fig. 12 is a schematic diagram showing an example of the magnetic flux density detected by the magnetic detection unit 43 from the tubular member 17 including the magnetic pole portions MA1 and MA2 shown in Fig. 11. The two graphs shown in Fig. 12 show the magnetic flux density BX and the magnetic flux density BY detected by the magnetic detection unit 43 in a case in which the flexible portion 10A is moved in the longitudinal direction X1 through the through-hole 41. In Fig. 12, a magnetic flux line from the positive pole region 17N to the negative pole region 17S is indicated by a broken line arrow.

As shown in the graph of Fig. 12, the magnetic flux density BX detected by the magnetic detection unit 43 has a positive value between each magnetic pole portion MA2 and the magnetic pole portions MA1 adjacent to the magnetic pole portion MA2 in the longitudinal direction X1, and has a negative value between each magnetic pole portion MA2 and the magnetic pole portion MA1 adjacent to the magnetic pole portion MA2 in the longitudinal direction X2. In addition, the magnetic flux density BY detected by the magnetic detection unit 43 has a positive value and a large absolute value in the vicinity of the position of each magnetic pole portion MA2, and has a negative value and a large absolute value in the vicinity of the position of each magnetic pole portion MA1.

Therefore, even in the configuration of the modification example shown in Fig. 11, it is possible to determine the movement state of the insertion part 10 by classifying the magnetic flux density BX shown in Fig. 12 into the high level and the low level as shown by a thick solid line, classifying the magnetic flux density BY shown in Fig. 12 into the high level and the low level as shown by a thick solid line, and monitoring the change in the combination thereof.

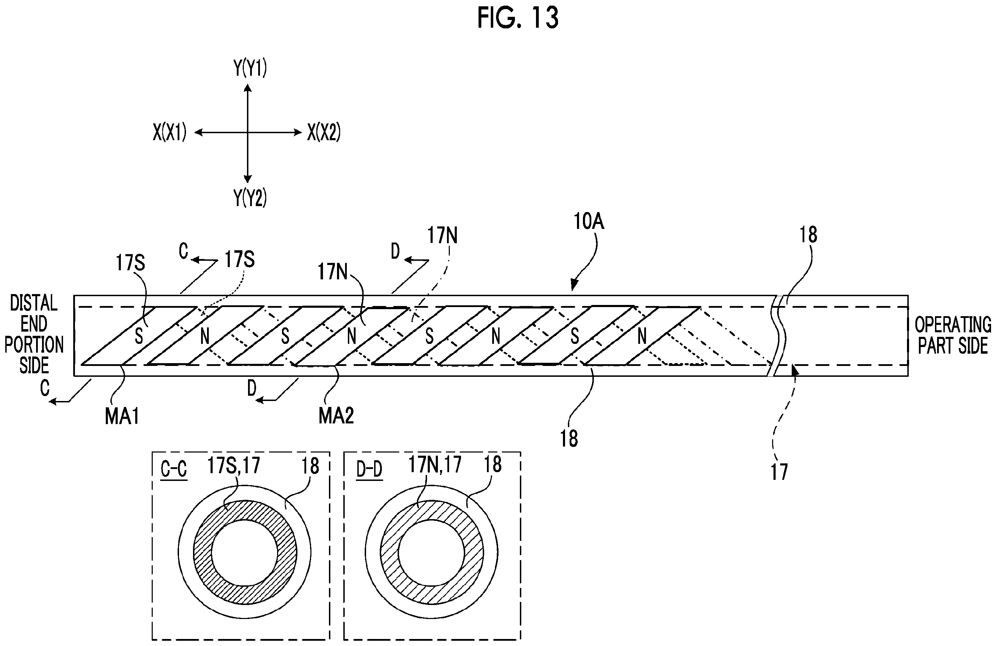

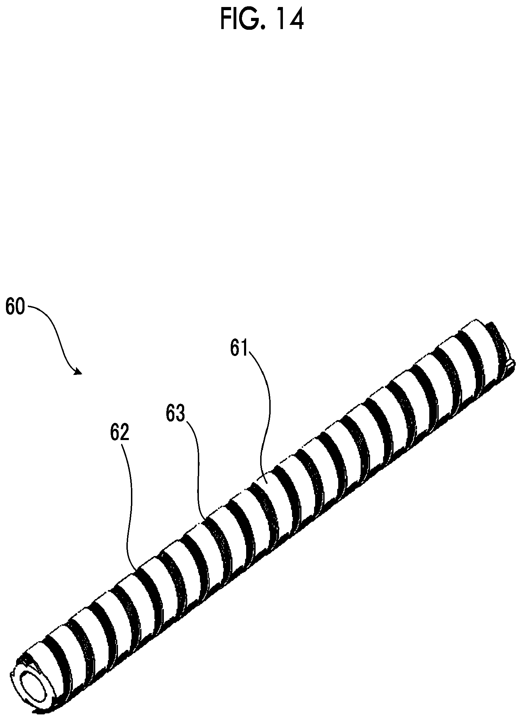

Fig. 13 is a schematic diagram showing a modification example of the magnetic pole portions MA1 and MA2 shown in Fig. 3. The modification example shown in Fig. 13 is different from that in Fig. 3 in that the negative pole region 17S formed on the magnetic pole portion MA1 and the positive pole region 17N formed on the magnetic pole portion MA2 are not formed perpendicular to the longitudinal direction X and formed at an angle, respectively, as viewed in the radial direction. It is possible to form such a magnetic pattern by using a magnetizer 60 as shown in Fig. 14.

The magnetizer 60 shown in Fig. 14 comprises a body part 61 having a tubular shape, and a first coil 62 and a second coil 63 disposed in a double groove on an outer peripheral surface of the body part 61. By disposing flexible portion 10A inside the body part 61, and passing the current through the first coil 62 and the second coil 63 in opposite directions to form the magnetic field in the body part 61, it is possible to form the magnetic pattern shown in Fig. 13. By using such a magnetizer 60, the magnetic pattern can be formed at once in the entire flexible portion 10A, and thus it is economical. In addition, since the magnetic pole portion MA can be easily and finely formed in the longitudinal direction X, it is possible to finely perform the determination of the movement distance of the insertion part 10.

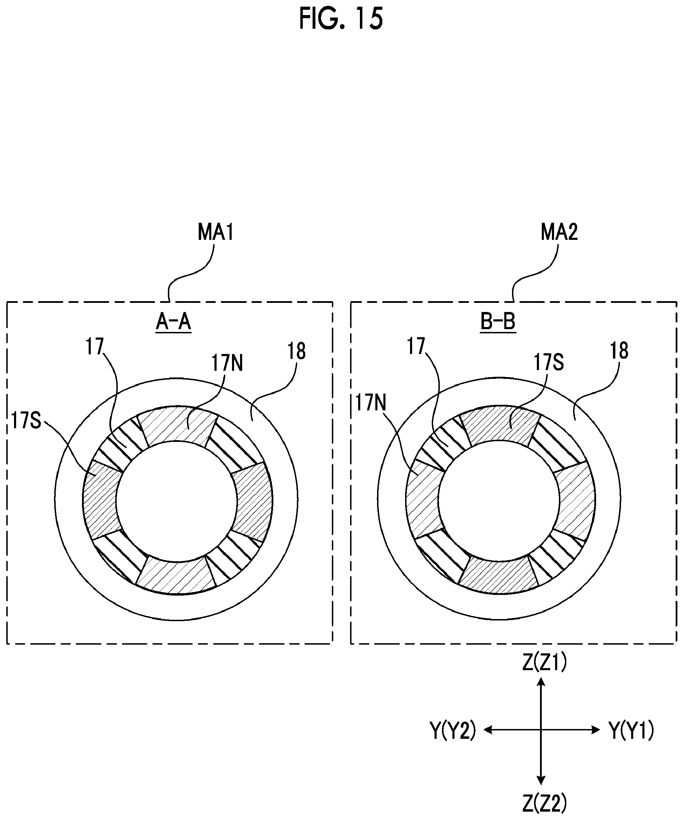

Fig. 15 is a schematic cross-sectional view showing a modification example of the magnetic pole portions MA1 and MA2 shown in Fig. 3 taken along the A-A arrow and the B-B arrow. In the modification example shown in Fig. 15, the magnetic pole portion MA1 has a configuration in which the negative pole region 17S and the positive pole region 17N are formed alternately and formed at an interval therebetween along the circumferential direction of the tubular member 17. Similarly, the magnetic pole portion MA2 has a configuration in which the negative pole region 17S and the positive pole region 17N are formed alternately and formed at an interval therebetween along the circumferential direction of the tubular member 17. The magnetic pole portion MA2 has a configuration in which the magnetic pole portion MA1 is rotated by 90 degrees around an axis center of the tubular member 17.

As shown in Fig. 15, in a state of being viewed in the longitudinal direction X, the positive pole region 17N in the magnetic pole portion MA1 and the negative pole region 17S in the magnetic pole portion MA2 are present at the same position in the circumferential direction of the tubular member 17. That is, in the tubular member 17, all the magnetic pole regions at the same position in the circumferential direction have a configuration in which the negative pole region 17S and the positive pole region 17N are alternately arranged in the longitudinal direction X. That is, in the tubular member 17, a first magnetic pattern in which the negative pole region 17S and the positive pole region 17N are alternately arranged along the longitudinal direction X with the negative pole region 17S at the beginning, and a second magnetic pattern in which the negative pole region 17S and the positive pole region 17N are alternately arranged along the longitudinal direction X with the positive pole region 17N at the beginning are alternately arranged and formed at an interval therebetween in the circumferential direction of the tubular member 17.

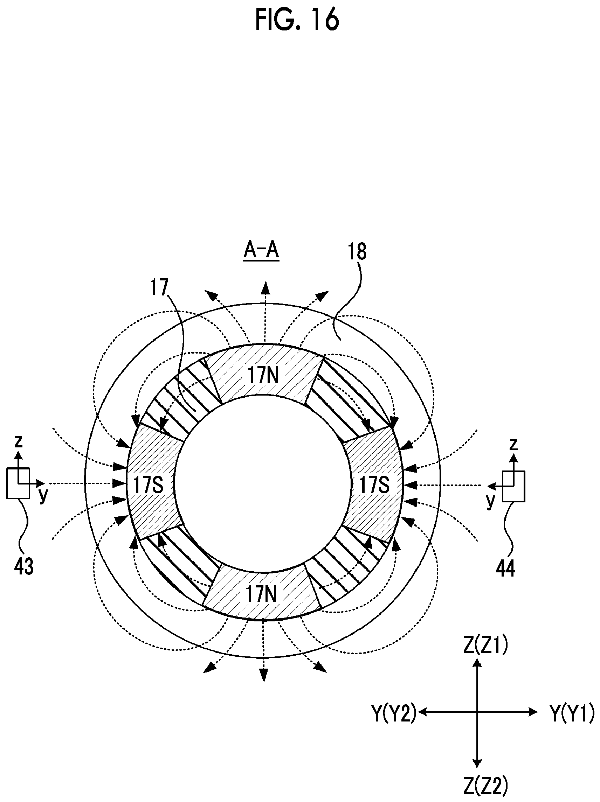

Fig. 16 is a diagram schematically showing a magnetic flux line generated in the magnetic pole portion MA1 having the configuration shown in Fig. 15. Fig. 16 shows the positions of the magnetic detection units 43 and 44 with respect to the flexible portion 10A in a case in which the flexible portion 10A passes through the through-hole 41.

In a state shown in Fig. 16, the magnetic flux density BY detected by the magnetic detection unit 43 has a large negative value. In a case in which the flexible portion 10A is rotated by 45 degrees counterclockwise from the state shown in Fig. 16, the magnetic flux density BY detected by the magnetic detection unit 43 has a value close to zero. In a case in which the flexible portion 10A is rotated by 90 degrees counterclockwise from the state shown in Fig. 16, the magnetic flux density BY detected by the magnetic detection unit 43 has a large positive value. In a case in which the flexible portion 10A is rotated by 135 degrees counterclockwise from the state shown in Fig. 16, the magnetic flux density BY detected by the magnetic detection unit 43 has a value close to zero. In a case in which the flexible portion 10A is rotated by 180 degrees counterclockwise from the state shown in Fig. 16, the magnetic flux density BY detected by the magnetic detection unit 43 has a large negative value. In a case in which the flexible portion 10A is rotated in the circumferential direction thereof in the through-hole 41 in this manner, the magnetic flux density BY detected by the magnetic detection unit 43 is equivalent to the magnetic flux density BY shown in Fig. 8. Similarly, in a case in which the flexible portion 10A is rotated in the circumferential direction thereof in the through-hole 41, the magnetic flux density BZ detected by the magnetic detection unit 43 is equivalent to the magnetic flux density BY shown in Fig. 8 and has a phase shifted by 90 degrees. Therefore, in a case in which the magnetic flux densities BY and BZ detected by the magnetic detection unit 43 are classified into the high level and the low level, respectively, these classification levels are equivalent to the thick solid lines of the magnetic flux density BY shown in Fig. 10 (it should be noted that the phases of the magnetic flux density BY and the magnetic flux density BZ are shifted by 90 degrees). Therefore, it is possible to derive a rotation direction and a rotation amount of the insertion part 10 by the combination of the classification levels.

In a case in which the endoscope 1 having the magnetic pattern having such a configuration is used, the processor 4P can determine a rotation state (rotation direction and rotation amount (rotation angle)) of the insertion part 10 in the circumferential direction in the same manner as the determination method of the movement state of the insertion part 10, by classifying each of the magnetic flux density BZ and the magnetic flux density BY into the plurality of pieces of information and monitoring the change in the combination of these pieces of information. It should be noted that, in the configuration shown in Fig. 15, since the first magnetic pattern and the second magnetic pattern extending in the longitudinal direction X are formed on the tubular member 17, it is possible to determine the movement state of the insertion part 10 based on the magnetic flux density BX and the magnetic flux density BY, as described above. In Fig. 15, each of the magnetic pole portions MA1 and the magnetic pole portions MA2 includes four magnetic pole regions arranged in the circumferential direction. However, a configuration may be adopted in which each of the magnetic pole portion MA1 and the magnetic pole portion MA2 includes two magnetic pole regions or an even number (six or more) of magnetic pole regions.

It should be noted that, even in the configuration shown in Fig. 15, it is preferable to obtain the arithmetic means of the magnetic flux densities BY and BZ detected by the magnetic detection unit 43 and the magnetic flux densities BY and BZ detected by the magnetic detection unit 44, classify the values of these two arithmetic means into the high level and the low level, respectively, and derive a rotation direction and a rotation amount of the insertion part 10 by the combination of the classification levels.

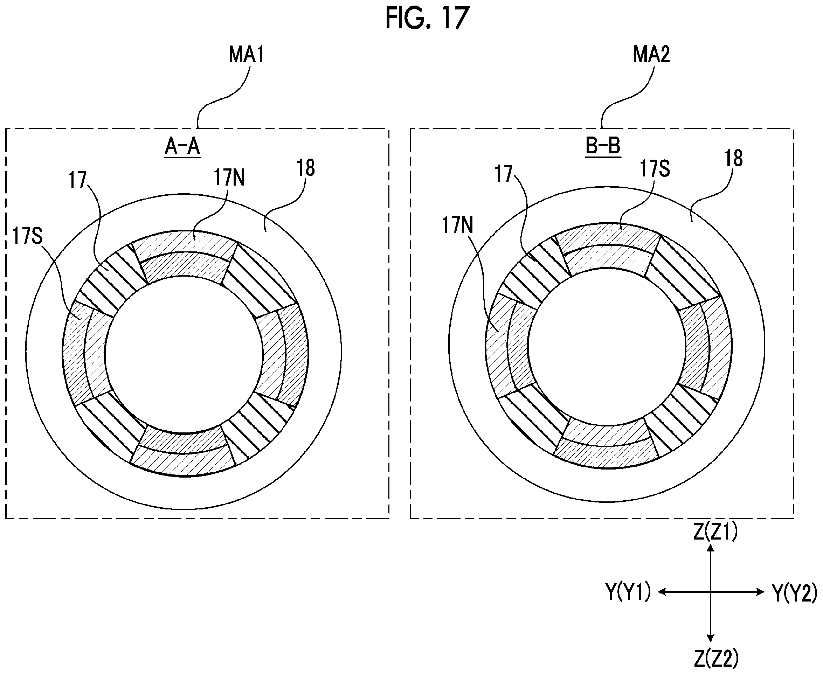

The configuration shown in Fig. 11 can also be applied to the configurations of the magnetic pole portions MA1 and MA2 shown in Fig. 15. Fig. 17 is a schematic cross-sectional view showing another modification example of the magnetic pole portions MA1 and MA2 shown in Fig. 3 taken along the A-A arrow and the B-B arrow. In the modification example shown in Fig. 17, in each of the magnetic pole portion MA1 and the magnetic pole portion MA2 shown in Fig. 15, the positive pole region 17N is changed to a two-layer structure of the positive pole region 17N formed on the outer peripheral side and the negative pole region 17S formed on an inner peripheral side, and the negative pole region 17S is changed to a two-layer structure in which the negative pole region 17S formed on the outer peripheral side and the positive pole region 17N formed on the inner peripheral side are formed. Even with the configuration shown in Fig. 17, the rotation state of the insertion part 10 can be determined based on the magnetic flux density BY and the magnetic flux density BZ.

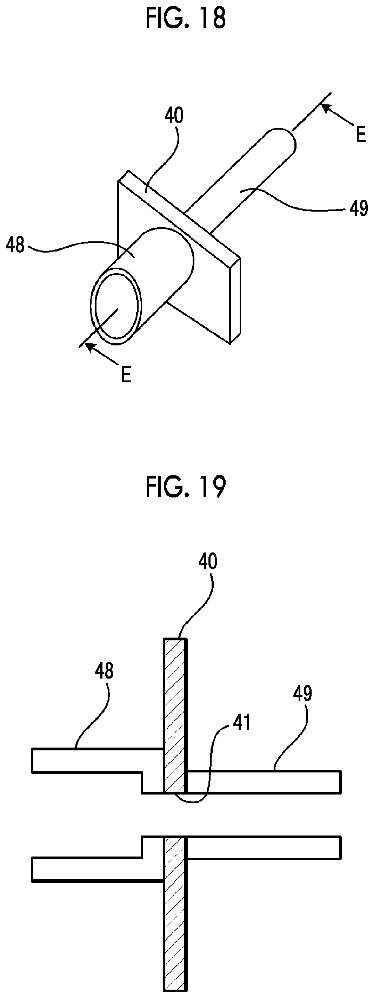

As described above, the detection unit 40 can also be integrally configured with the insertion assisting member of the endoscope 1. Fig. 18 is a perspective view schematically showing a configuration example in which the detection unit 40 and the insertion assisting member are integrated. Fig. 19 is a schematic cross-sectional view taken along an E-E arrow in Fig. 18.

In the example shown in Fig. 18, the first member 48 having a tubular shape is fixed to one surface of the detection unit 40, and the second member 49 having a tubular shape is fixed to the other surface of the detection unit 40. An outer diameter of the second member 49 is less than an outer diameter of the first member 48. The inside of the first member 48 and the inside of the second member 49 communicate with each other through the through-hole 41 of the detection unit 40. During the endoscopy, the insertion of the endoscope 1 into the first member 48 in a state in which the second member 49 is inserted into the anus assists the insertion of the endoscope 1 into the body.

It should be noted that the detection unit 40 may be integrally formed with a mouthpiece-type insertion assisting member that is held in the mouth. In addition, the detection unit 40 may be formed integrally with the pants for endoscopy, or may be configured to be detachable from the pants for endoscopy.

The technology of the present disclosure is not limited to the above description, and can be appropriately changed as described below.

For example, the endoscope 1 may be inserted into the body through the mouth or the nose of the subject 50.

In this case, the detection unit 40 need only have a shape that can be attached to the mouth or the nose of the subject 50.

The tubular member 17 has the configuration in which the first member 14 and the second member 15 are provided, and each of the first member 14 and the second member 15 contains the magnetizable austenite stainless steel, but one of the first member 14 or the second member 15 may be made of a non-magnetizable material. That is, the magnetic pattern does not have to be formed on one of the first member 14 or the second member 15. Even in such a case, since the magnetic flux densities BX, BY, and BZ can be detected from the tubular member 17, it is possible to determine the movement state and the rotation state of the insertion part 10.

The detection unit 40 is provided with one set of the magnetic detection unit 43 and the magnetic detection unit 44 facing each other with the through-hole 41 interposed therebetween, but the detection unit 40 may be provided with a plurality of such sets. For example, in Fig. 6, the set of the magnetic detection unit 43 and the magnetic detection unit 44 that are arranged in the direction z with the center CP of the through-hole 41 interposed therebetween may be added. In this case, based on each of the magnetic flux densities BX, BY, and BZ detected by the set of the magnetic detection unit 43 and the magnetic detection unit 44 arranged in the radial direction y, and the magnetic flux densities BX, BY, and BZ detected by the set of the magnetic detection unit 43 and the magnetic detection unit 44 arranged in the direction z, the movement state and the rotation state of the insertion part 10 may be determined, the determination results may be integrated to finally determine the movement state and the rotation state of the insertion part 10, and notification may be given to the operator.

The technology of the present disclosure can also be applied to the treatment tool that has an elongated shape and is used by being inserted into the endoscope 1. That is, by forming the magnetic pattern as described above on the treatment tool, it is possible to determine the movement state of the treatment tool in the longitudinal direction and the rotation state of the treatment tool in the circumferential direction. In forming the magnetic pattern on the treatment tool, for example, the magnetic pattern need only be magnetized on a wire for opening and closing the forceps, a close attachment spring covering the wire, or the like. Also, in the guide wire, a metal wire constituting the insertion part need only be magnetized. It should be noted that, in a case in which the movement state or the rotation state of the treatment tool is determined, the magnetic detection unit 43 and the magnetic detection unit 44 may be disposed in the vicinity of an insertion port of the treatment tool provided in the operating part 11, or may be disposed in the vicinity of a delivery port of the treatment tool provided in the distal end portion 10C. In a case in which the magnetic detection unit 43 and the magnetic detection unit 44 are provided in the insertion port of the operating part 11, by storing a distance from the insertion port to the delivery port of the treatment tool at the distal end of the endoscope 1 in the memory, it is possible to derive a protrusion length of the treatment tool protruding from the delivery port of the treatment tool based on the detection results of the magnetic detection unit 43 and the magnetic detection unit 44.

By adopting the technology of the present disclosure, for example, even in a case of an overtube, such as a double balloon or a single balloon, attached to the outer periphery of the insertion part 10, similarly, it is possible to determine the insertion length into the body, determine the movement direction, determine the movement distance, and the like.

As described above, in the tubular member 17, the two types of magnetic pole regions are alternately arranged in the longitudinal direction to form the magnetic pattern, and the movement state of the insertion part 10 in the longitudinal direction is determined based on the combination of the classification levels of the magnetic information in the two directions detected from the magnetic pattern. However, the two types of magnetic pole regions formed on the tubular member 17 do not have to be alternately arranged in the longitudinal direction. Even in such a case, the movement state of the insertion part 10 in the longitudinal direction can be determined based on the combination of the classification levels of the magnetic information in the two directions detected from the magnetic pattern.

In addition, as a modification example, the movement state of the insertion part 10 in the longitudinal direction may be determined by forming a pattern more complicated than the magnetic pattern on the tubular member 17 and detecting the pattern by the magnetic detection units 43 and 44. Specifically, a table in which each position of the tubular member 17 in the longitudinal direction and the magnetic flux density BX or the magnetic flux density BY (classification level) detected at each position are associated with each other may be stored in a memory, and the processor 4P may classify the magnetic flux density BX or the magnetic flux density BY detected by the magnetic detection unit 43 to acquire the classification level, and may acquire the information on the position corresponding to the classification level from the table to determine the insertion length of the insertion part 10. As a result, the insertion length of the insertion part 10 can be finely determined. In addition, the magnetic detection units 43 and 44 can detect the magnetic flux densities in one direction, so that the cost can be reduced.

### Explanation of References

1: endoscope
MA, MA1, MA2: magnetic pole portion
4P: processor
4: processor device
5: light source device
6: input unit
7: display unit
10A: flexible portion
10B: bendable portion
10C: distal end portion
10: insertion part
11: operating part
12: angle knob
13A, 13B: connector portion
13: universal cord
14: first member
15a: metal strip
15: second member
16A, 16B: cap
17N: positive pole region
17S: negative pole region
17: tubular member
18A: inner resin layer
18B: outer resin layer
18: outer skin layer
19: coating layer
40: insertion assisting member
42: housing
42A: body part
42B: lid part
42a: flat plate portion
42b: side wall portion
42c: inner wall portion
41A, 41B. 41: through-hole
43, 44: magnetic detection unit
45: communication chip
46: storage battery
47: power receiving coil
48: first member
49: second member
50A: anus
50: subject
60: magnetizer
61: body part
62: first coil
63: second coil
100: endoscope apparatus
200: endoscope system
300: magnetic field generation device

## Claims

1. A system comprising:
a detection unit (40) comprising a housing (42) having a through-hole (41) therethrough and a magnetic detection unit (43, 44) in the housing;
an instrument that has an elongated shape and is used by being relatively moved with respect to the magnetic detection unit; and
a processor (4P),
wherein the instrument includes a member that extends in a longitudinal direction and has a magnetic pattern formed along the longitudinal direction,
the magnetic detection unit (43, 44) detects a first magnetic flux density in a first direction and a second magnetic flux density in a second direction intersecting the first direction at a plurality of positions along the longitudinal direction of the member, **characterised in that**
the processor determines a movement state of the instrument in the longitudinal direction based on the first magnetic flux density and the second magnetic flux density which are detected by the magnetic detection unit as the instrument passes through the through-hole (41); wherein the first direction is a direction different from a radial direction and a circumferential direction of the instrument, and
the second direction is a direction different from the circumferential direction and the longitudinal direction of the instrument.

2. The system according to claim 1,
wherein the first direction is the longitudinal direction, and
the second direction is the radial direction.

3. The system according to claim 1 or 2,
wherein the processor (4P) determines a movement direction of the instrument in the longitudinal direction based on the first magnetic flux density and the second magnetic flux density.

4. The system according to any preceding claim,
wherein the magnetic pattern includes a pattern in which two types of magnetic pole regions are alternately arranged along the longitudinal direction.

5. The system according to any preceding claim,
wherein the magnetic pattern includes a pattern in which two types of magnetic pole regions are arranged along the longitudinal direction, and
the processor determines a movement amount of the instrument in the longitudinal direction with a resolution finer than an interval between the two types of magnetic pole regions adjacent to each other, based on the first magnetic flux density and the second magnetic flux density.

6. The system according to claim 5,
wherein the processor
classifies the first magnetic flux density into a plurality of pieces of information according to magnitude thereof, and classifies the second magnetic flux density into a plurality of pieces of information according to magnitude thereof, and
determines the movement state of the instrument in the longitudinal direction based on a combination of any of the plurality of pieces of information obtained by classifying the first magnetic flux density and any of the plurality of pieces of information obtained by classifying the second magnetic flux density.

7. The system according to claim 6,
wherein the processor
classifies one of the first magnetic flux density or the second magnetic flux density into at least three pieces of information according to the magnitude thereof, and
determines the movement state of the instrument in the longitudinal direction based on a combination of any of the plurality of pieces of information obtained by classifying the other of the first magnetic flux density or the second magnetic flux density and any of the at least three pieces of information obtained by classifying the one of the first magnetic flux density or the second magnetic flux density,
the magnetic pattern includes a pattern in which the two types of magnetic pole regions are alternately arranged along the longitudinal direction,
one of the first magnetic flux density or the second magnetic flux density is classified into first information at a position of one of the two types of magnetic pole regions, is classified into second information at a position of the other of the two types of magnetic pole regions, and is classified into third information at a position between the two types of magnetic pole regions, and
the other of the first magnetic flux density or the second magnetic flux density is classified into fourth information at a position between the magnetic pole region and the magnetic pole region adjacent to the magnetic pole region on one side in the longitudinal direction, and is classified into fifth information at a position between the magnetic pole region and the magnetic pole region adjacent to the magnetic pole region on the other side in the longitudinal direction.

8. The system according to claim 6,
wherein the processor classifies the first magnetic flux density based on a first threshold value related to magnitude of a magnetic flux density, and classifies the second magnetic flux density based on a second threshold value related to magnitude of a magnetic flux density, and
the processor determines the first threshold value and the second threshold value based on the magnetic flux density detected from the member by the magnetic detection unit.

9. The system according to any preceding claim,
wherein the magnetic detection unit further detects a third magnetic flux density in a third direction different from a radial direction and the longitudinal direction of the instrument, and
the processor determines a rotation state of the instrument in a circumferential direction based on the second magnetic flux density and the third magnetic flux density which are detected by the magnetic detection unit.

10. The system according to claim 9,
wherein the magnetic pattern has a configuration in which a plurality of magnetic pole portions in which two types of magnetic pole regions are alternately arranged along the circumferential direction of the instrument are arranged in the longitudinal direction, and
the processor determines a rotation amount of the instrument in the circumferential direction with a resolution finer than an interval between the two types of magnetic pole regions adjacent to each other in the circumferential direction, based on the second magnetic flux density and the third magnetic flux density.

11. The system according to any preceding claim,
wherein the magnetic detection unit includes a first magnetic detection unit and a second magnetic detection unit which are arranged along a circumferential direction of the instrument,
the first magnetic detection unit and the second magnetic detection unit each detect a third magnetic flux density in a third direction different from a radial direction and the longitudinal direction of the instrument, and
the processor determines a rotation state of the instrument in the circumferential direction based on the third magnetic flux density detected by the first magnetic detection unit and the third magnetic flux density detected by the second magnetic detection unit.

12. The system according to any preceding claim,
wherein the instrument is an insertion part (10), which is inserted into a body, of a medical device including the insertion part.

13. The system according to claim 12,
wherein the medical device is an endoscope (100).

14. The system according to claim 13,
wherein the insertion part of the endoscope includes a distal end portion (10C) and a flexible portion (10A), and
the member is provided in the flexible portion.

## Patentansprüche

1. System, umfassend:
eine Detektionseinheit (40), die ein Gehäuse (42), das ein Durchgangsloch (41) dadurch aufweist, und eine Magnetdetektionseinheit (43, 44) in dem Gehäuse umfasst;
ein Instrument, das eine längliche Form aufweist und verwendet wird, indem es relativ in Bezug auf die Magnetdetektionseinheit bewegt wird; und
einen Prozessor (4P),
wobei das Instrument ein Element, das sich in einer Längsrichtung erstreckt und ein Magnetmuster, das entlang der Längsrichtung gebildet ist, aufweist, enthält,
die Magnetdetektionseinheit (43, 44) eine erste Magnetflussdichte in einer ersten Richtung und eine zweite Magnetflussdichte in einer zweiten Richtung, die die erste Richtung an mehreren Positionen entlang der Längsrichtung des Elements schneidet, detektiert, **dadurch gekennzeichnet, dass**
der Prozessor einen Bewegungszustand des Instruments in der Längsrichtung auf der Grundlage der ersten Magnetflussdichte und der zweiten Magnetflussdichte, die von der Magnetdetektionseinheit detektiert werden, während das Instrument das Durchgangsloch (41) passiert, bestimmt; wobei
die erste Richtung eine Richtung, die sich von einer radialen Richtung und einer Umfangsrichtung des Instruments unterscheidet, ist, und
die zweite Richtung eine Richtung, die sich von der Umfangsrichtung und der Längsrichtung des Instruments unterscheidet, ist.

2. System nach Anspruch 1,
wobei die erste Richtung die Längsrichtung ist, und
die zweite Richtung die Radialrichtung ist.

3. System nach Anspruch 1 oder 2,
wobei der Prozessor (4P) eine Bewegungsrichtung des Instruments in der Längsrichtung auf der Grundlage der ersten Magnetflussdichte und der zweiten Magnetflussdichte bestimmt.

4. System nach einem der vorhergehenden Ansprüche,
wobei das Magnetmuster ein Muster, bei dem zwei Typen von Magnetpolbereichen entlang der Längsrichtung abwechselnd angeordnet sind, enthält.

5. System nach einem der vorhergehenden Ansprüche,
wobei das Magnetmuster ein Muster, bei dem zwei Typen von Magnetpolbereichen entlang der Längsrichtung angeordnet sind, enthält, und
der Prozessor einen Bewegungsbetrag des Instruments in der Längsrichtung mit einer Auflösung, die feiner als ein Intervall zwischen den beiden Typen von Magnetpolbereichen, die zueinander benachbart sind, ist, auf der Grundlage der ersten Magnetflussdichte und der zweiten Magnetflussdichte bestimmt.

6. System nach Anspruch 5,
wobei der Prozessor
die erste Magnetflussdichte in mehrere Informationen gemäß Größe davon klassifiziert und die zweite Magnetflussdichte in mehrere Informationen gemäß Größe davon klassifiziert, und
den Bewegungszustand des Instruments in der Längsrichtung auf der Grundlage einer Kombination aus beliebigen von den mehreren Informationen, die durch Klassifizieren der ersten Magnetflussdichte erhalten wurden, und beliebigen von den mehreren Informationen, die durch Klassifizieren der zweiten Magnetflussdichte erhalten wurden, bestimmt.

7. System nach Anspruch 6,
wobei der Prozessor
eine von der ersten Magnetflussdichte und der zweiten Magnetflussdichte mindestens in drei Informationen gemäß der Größe davon klassifiziert, und
den Bewegungszustand des Instruments in der Längsrichtung auf der Grundlage einer Kombination aus einer beliebigen von den mehreren Informationen, die durch Klassifizieren der anderen von der ersten Magnetflussdichte und der zweiten Magnetflussdichte erhalten wurden, und einer beliebigen von den mindestens drei Informationen, die durch Klassifizieren der einen von der ersten Magnetflussdichte oder der zweiten Magnetflussdichte erhalten wurden, bestimmt,
das Magnetmuster ein Muster, bei dem die zwei Typen von Magnetpolbereichen entlang der Längsrichtung abwechselnd angeordnet sind, enthält,
eine von der ersten Magnetflussdichte und der zweiten Magnetflussdichte an einer Position eines der beiden Typen von Magnetpolbereichen in erste Informationen klassifiziert wird, an einer Position des anderen der beiden Typen von Magnetpolbereichen in zweite Informationen klassifiziert wird und an einer Position zwischen den beiden Typen von Magnetpolbereichen in dritte Informationen klassifiziert wird, und
das andere der ersten Magnetflussdichte und der zweiten Magnetflussdichte an einer Position zwischen dem Magnetpolbereich und dem Magnetpolbereich, der zu dem Magnetpolbereich auf einer Seite in der Längsrichtung benachbart ist, in vierte Informationen klassifiziert wird und an einer Position zwischen dem Magnetpolbereich und dem Magnetpolbereich, der zu dem Magnetpolbereich auf der anderen Seite in der Längsrichtung benachbart ist, in fünfte Informationen klassifiziert wird.

8. System nach Anspruch 6,
wobei der Prozessor die erste Magnetflussdichte auf der Grundlage eines ersten Schwellenwerts, der sich auf Größe einer Magnetflussdichte bezieht, klassifiziert und die zweite Magnetflussdichte auf der Grundlage eines zweiten Schwellenwerts, der sich auf Größe einer Magnetflussdichte bezieht, klassifiziert, und
der Prozessor den ersten Schwellenwert und den zweiten Schwellenwert auf der Grundlage der von dem Element durch die Magnetdetektionseinheit detektierten Magnetflussdichte bestimmt.

9. System nach einem der vorhergehenden Ansprüche,
wobei die Magnetdetektionseinheit ferner eine dritte Magnetflussdichte in einer dritten Richtung, die sich von einer Radialrichtung und der Längsrichtung des Instruments unterscheidet, detektiert, und
der Prozessor einen Drehzustand des Instruments in einer Umfangsrichtung auf der Grundlage der zweiten Magnetflussdichte und der dritten Magnetflussdichte, die von der Magnetdetektionseinheit detektiert werden, bestimmt.

10. System nach Anspruch 9,
wobei das Magnetmuster eine Konfiguration, bei der mehrere Magnetpolabschnitte, in denen zwei Typen von Magnetpolbereichen entlang der Umfangsrichtung des Instruments abwechselnd angeordnet sind, in der Längsrichtung angeordnet sind, aufweist, und
der Prozessor einen Drehbetrag des Instruments in der Umfangsrichtung mit einer Auflösung, die feiner als ein Intervall zwischen den beiden Typen von Magnetpolbereichen, die in der Umfangsrichtung zueinander benachbart sind, ist, auf der Grundlage der zweiten Magnetflussdichte und der dritten Magnetflussdichte bestimmt.

11. System nach einem der vorhergehenden Ansprüche,
wobei die Magnetdetektionseinheit eine erste Magnetdetektionseinheit und eine zweite Magnetdetektionseinheit, die entlang einer Umfangsrichtung des Instruments angeordnet sind, enthält,
die erste Magnetdetektionseinheit und die zweite Magnetdetektionseinheit jeweils eine dritte Magnetflussdichte in einer dritten Richtung, die sich von einer Radialrichtung und der Längsrichtung des Instruments unterscheidet, detektieren, und
der Prozessor einen Drehzustand des Instruments in der Umfangsrichtung auf der Grundlage der dritten Magnetflussdichte, die von der ersten Magnetdetektionseinheit detektiert wurde, und der dritten Magnetflussdichte, die von der zweiten Magnetdetektionseinheit detektiert wurde, bestimmt.

12. System nach einem der vorhergehenden Ansprüche,
wobei das Instrument ein Einführteil (10), der in einen Körper eingeführt wird, von einer medizinischen Vorrichtung, die den Einführteil enthält, ist.

13. System nach Anspruch 12,
wobei die medizinische Vorrichtung ein Endoskop (100) ist.

14. System nach Anspruch 13,
wobei der Einführteil des Endoskops einen distalen Endabschnitt (10C) und einen flexiblen Abschnitt (10A) enthält, und
das Element in dem flexiblen Abschnitt vorgesehen ist.

## Revendications

1. Système comprenant :
une unité de détection (40) comprenant un boîtier (42) ayant un trou traversant (41) et une unité de détection magnétique (43, 44) dans le boîtier ;
un instrument qui a une forme allongée et est utilisé en étant déplacé relativement par rapport à l'unité de détection magnétique ; et
un processeur (4P),
dans lequel l'instrument inclut un élément qui s'étend dans une direction longitudinale et présentant un motif magnétique formé le long de la direction longitudinale,
l'unité de détection magnétique (43, 44) détecte une première densité de flux magnétique dans une première direction et une deuxième densité de flux magnétique dans une deuxième direction croisant la première direction à une pluralité de positions le long de la direction longitudinale de l'élément, **caractérisé en ce que**
le processeur détermine un état de déplacement de l'instrument dans la direction longitudinale sur la base de la première densité de flux magnétique et de la deuxième densité de flux magnétique qui sont détectées par l'unité de détection magnétique lorsque l'instrument passe à travers le trou traversant (41) ; dans lequel
la première direction est une direction différente d'une direction radiale et d'une direction circonférentielle de l'instrument, et
la deuxième direction est une direction différente de la direction circonférentielle et de la direction longitudinale de l'instrument.

2. Système selon la revendication 1,
dans lequel la première direction est la direction longitudinale, et
la deuxième direction est la direction radiale.

3. Système selon la revendication 1 ou la revendication 2,
dans lequel le processeur (4P) détermine une direction de déplacement de l'instrument dans la direction longitudinale sur la base de la première densité de flux magnétique et de la deuxième densité de flux magnétique.

4. Système selon l'une quelconque des revendications précédentes,
dans lequel le motif magnétique inclut un motif dans lequel deux types de régions de pôle magnétique sont disposées de manière alternée le long de la direction longitudinale.

5. Système selon l'une quelconque des revendications précédentes,
dans lequel le motif magnétique inclut un motif dans lequel deux types de régions de pôle magnétique sont disposées le long de la direction longitudinale, et
le processeur détermine une quantité de déplacement de l'instrument dans la direction longitudinale avec une résolution plus fine qu'un intervalle entre les deux types de régions de pôle magnétique adjacentes l'une à l'autre, sur la base de la première densité de flux magnétique et de la deuxième densité de flux magnétique.

6. Système selon la revendication 5,
dans lequel le processeur
classe la première densité de flux magnétique en une pluralité d'éléments d'informations selon son intensité, et classe la deuxième densité de flux magnétique en une pluralité d'éléments d'informations selon son intensité, et
détermine l'état de déplacement de l'instrument dans la direction longitudinale sur la base d'une combinaison de l'une quelconque de la pluralité d'éléments d'informations obtenus par classification de la première densité de flux magnétique et de l'une quelconque de la pluralité d'éléments d'informations obtenus par classification de la deuxième densité de flux magnétique.

7. Système selon la revendication 6,
dans lequel le processeur
classe l'une de la première densité de flux magnétique ou de la deuxième densité de flux magnétique en au moins trois éléments d'informations selon son intensité, et
détermine l'état de déplacement de l'instrument dans la direction longitudinale sur la base d'une combinaison de l'une quelconque de la pluralité d'éléments d'informations obtenus par classification de l'autre de la première densité de flux magnétique ou de la deuxième densité de flux magnétique et de l'une quelconque d'au moins trois éléments d'informations obtenus par classification de l'une de la première densité de flux magnétique ou de la deuxième densité de flux magnétique,
le motif magnétique inclut un motif dans lequel les deux types de régions de pôle magnétique sont disposées de manière alternée le long de la direction longitudinale, l'une de la première densité de flux magnétique ou de la deuxième densité de flux magnétique est classée en premières informations à une position d'un des deux types de régions de pôle magnétique, est classée en deuxièmes informations à une position de l'autre des deux types de régions de pôle magnétique, et est classée en troisièmes informations à une position entre les deux types de régions de pôle magnétique, et
l'autre de la première densité de flux magnétique ou de la deuxième densité de flux magnétique est classé en quatrièmes informations à une position entre la région de pôle magnétique et la région de pôle magnétique adjacente à la région de pôle magnétique d'un côté dans la direction longitudinale, et est classé en cinquièmes informations à une position entre la région de pôle magnétique et la région de pôle magnétique adjacente à la région de pôle magnétique de l'autre côté dans la direction longitudinale.

8. Système selon la revendication 6,
dans lequel le processeur classe la première densité de flux magnétique sur la base d'une première valeur seuil liée à l'intensité d'une densité de flux magnétique, et classe la deuxième densité de flux magnétique sur la base d'une deuxième valeur seuil liée à l'intensité d'une densité de flux magnétique, et
le processeur détermine la première valeur seuil et la deuxième valeur seuil sur la base de la densité de flux magnétique détectée à partir de l'élément par l'unité de détection magnétique.

9. Système selon l'une quelconque des revendications précédentes,
dans lequel l'unité de détection magnétique détecte en outre une troisième densité de flux magnétique dans une troisième direction différente d'une direction radiale et de la direction longitudinale de l'instrument, et
le processeur détermine un état de rotation de l'instrument dans une direction circonférentielle sur la base de la deuxième densité de flux magnétique et de la troisième densité de flux magnétique qui sont détectées par l'unité de détection magnétique.

10. Système selon la revendication 9,
dans lequel le motif magnétique présente une configuration dans laquelle une pluralité de parties de pôle magnétique dans lesquelles deux types de régions de pôle magnétique sont disposées de manière alternée le long de la direction circonférentielle de l'instrument sont disposées dans la direction longitudinale, et
le processeur détermine une quantité de rotation de l'instrument dans la direction circonférentielle avec une résolution plus fine qu'un intervalle entre les deux types de régions de pôle magnétique adjacentes l'une à l'autre dans la direction circonférentielle, sur la base de la deuxième densité de flux magnétique et de la troisième densité de flux magnétique.

11. Système selon l'une quelconque des revendications précédentes,
dans lequel l'unité de détection magnétique inclut une première unité de détection magnétique et une deuxième unité de détection magnétique qui sont disposées le long d'une direction circonférentielle de l'instrument,
la première unité de détection magnétique et la deuxième unité de détection magnétique détectent chacune une troisième densité de flux magnétique dans une troisième direction différente d'une direction radiale et de la direction longitudinale de l'instrument, et
le processeur détermine un état de rotation de l'instrument dans la direction circonférentielle sur la base de la troisième densité de flux magnétique détectée par la première unité de détection magnétique et de la troisième densité de flux magnétique détectée par la deuxième unité de détection magnétique.

12. Système selon l'une quelconque des revendications précédentes,
dans lequel l'instrument est une partie d'insertion (10), qui est insérée dans un corps, d'un dispositif médical incluant la partie d'insertion.

13. Système selon la revendication 12,
dans lequel le dispositif médical est un endoscope (100).

14. Système selon la revendication 13,
dans lequel la partie d'insertion de l'endoscope inclut une partie d'extrémité distale (10C) et une partie flexible (10A), et
l'élément est prévu dans la partie flexible.
